# EUROPEAN PATENT APPLICATION

(11) **EP 3 388 430 A1**
(43) Date of publication of application: **17.10.2018**
(21) Application number: 17165807.3
(22) Date of filing: 10.04.2017
(51) Int. Cl.: C07D 417/12, C07D 277/56, A61K 31/426, A61P 35/00

(54) **COMPOUND, COMPOUND FOR USE IN THE TREATMENT OF A PATHOLOGICAL CONDITION, A PHARMACEUTICAL COMPOSITION AND A METHOD FOR PREPARING SAID COMPOUND**

(71) Applicant: Technische Universität München, 80333 München (DE); Deutsches Krebsforschungszentrum Heidelberg Stiftung des öffentlichen Rechts (DKFZ), 69120 Heidelberg (DE)
(72) Inventor: Kuster, Bernhard, 85417 Marzling (DE); Médard, Guillaume, 80804 München (DE); Heinzlmeir, Stephanie, 85229 Markt Indersdorf (DE); Schwalbe, Harald, 61449 Steinbach (DE); Kudlinzki, Denis, 99998 Körner (DE)
(74) Representative: Dr. Gassner & Partner mbB

(57) **Abstract**

The invention concerns a compound, wherein the compound is a substance according to the following formula (I) 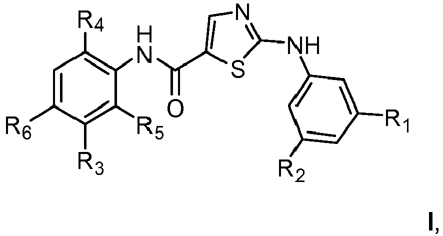 wherein R₁ is 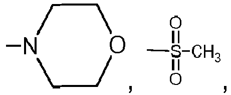 -SO₃H, 1-methyl-4,5-dihydro-1H-imidazol-2-yl, 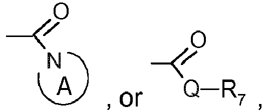 wherein the ring A is a pyrrolidine, piperidine, morpholine, or imidazole residue or a substituted pyrrolidine, piperidine, morpholine, or imidazole residue, Q is -NH-, -NH-alkyl-, -O-, or -S- and R₇ is -H, a substituted or non-substituted pyrrolidine, piperidine, morpholine, triazine, or imidazole residue, or a substituted or non-substituted alkylpyrrolidine, alkylpiperidine, alkylmorpholine, alkyltriazine, or alkylimidazole residue, alkyl, alkylester, alkenyl, alkenylester, alkynyl, alkynylester, aminoalkyl, cyanyl, -NO₂, methylsulfonyl residue, or a substituted or non-substituted form of any of the following residues: cycloalkyl, cycloalkylalkyl, cycloalkenyl, cycloalkenylalkyl, bicycloalkyl, bicycloalkylalkyl, bicycloalkenyl, bicycloalkenylalkyl, or another bicyclic residue, aryl, aminoaryl, heteroaryl, heteroaminoaryl, cyclyl, aminocyclyl, heterocyclyl, heteroaminocyclyl, aralkyl, aminoaralkyl, heterocycloalkyl, or heteroaminocycloalkyl,
R₂ is 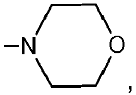 -H, -Cl, -Br, -CF₃, -CH=CH₂ or another alkenyl, -C=CH or another alkynyl, phenyl or substituted phenyl, triazolyl or another heteroaryl, substituted triazolyl or another substituted heteroaryl, a residue of 9-BBN (9-borabicyclo[3.3.1]nonane), OBBD (9-oxa-10-borabicyclo[3.3.2]decane), trifluoroborate, boronic acid, or an ester of boronic acid and pinacol, methyliminodiacetic acid (MIDA), catechol, pinane diol, ethylene glycol, or propane diol or another boronic acid ester, 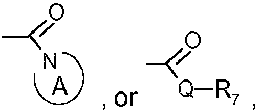 wherein the ring A is a pyrrolidine, piperidine, morpholine, or imidazole residue or a substituted pyrrolidine, piperidine, morpholine, or imidazole residue, Q is -NH-, -NH-alkyl-, -O-, or -S- and R₇ is -H, a substituted or non-substituted pyrrolidine, piperidine, morpholine, triazine, or imidazole residue, or a substituted or non-substituted alkylpyrrolidine, alkylpiperidine, alkylmorpholine, alkyltriazine, or alkylimidazole residue, alkyl, alkylester, alkenyl, alkenylester, alkynyl, alkynylester, aminoalkyl, cyanyl, -NO₂, methylsulfonyl residue, or a substituted or non-substituted form of any of the following residues: cycloalkyl, cycloalkylalkyl, cycloalkenyl, cycloalkenylalkyl, bicycloalkyl, bicycloalkylalkyl, bicycloalkenyl, bicycloalkenylalkyl, or another bicyclic residue, aryl, aminoaryl, heteroaryl, heteroaminoaryl, cyclyl, aminocyclyl, heterocyclyl, heteroaminocyclyl, aralkyl, aminoaralkyl, heterocycloalkyl, or heteroaminocycloalkyl,
R₃ is -H or -CH₃,
R₄ is -H, -Cl, -Br, -CH₃, -CH(CH₃)₂ or -C(CH₃)₃,
R₅ is -H, -CH₃, -CH(CH₃)₂ or -C(CH₃)₃, and
R₆ is -H or -CH₃,
or a salt of said substance.

## Description

The invention concerns a compound, said compound for use in the treatment of a pathologically increased activity of a kinase, a pharmaceutical composition and a method for preparing the compound.

From EP 1 169 038 B9 cyclic compounds and salts thereof, such compounds for use in treating protein tyrosine kinase associated disorders such as immunologic and oncologic disorders, and pharmaceutical compositions containing such compounds are known. All these compounds comprise an aminothiazole core structure.

WO 2008/150446 A1 concerns protein kinase inhibitors such as Src-protein kinase inhibitors as well as a medical use of the inhibitors, e. g. for the treatment of cancer.

WO 2005/000298 A2 discloses aminoaryl substituted 5-membered heterocyclic compounds which have cytokine inhibitory activity and the use of said compounds for treating conditions associated with p38α and β kinases and for treating p38 kinase-associated conditions.

Chang, Q. et al., British Journal of Cancer (2008) 99, pages 1074 to 1082 disclose effects of the multi-targeted kinase inhibitor dasatinib on EphA2 receptor tyrosine kinase activity and downstream signaling in pancreatic cancer. Dasatinib was mainly developed for Bcr-Abl and Src family kinases as inhibitor. The authors of the publication found that EphA2 receptor tyrosine kinase was inhibited directly by dasatinib in a dose-dependent manner.

The problem to be solved by the present invention is to provide an alternative compound and in particular a compound for use in the treatment of a pathologically increased activity of a protein tyrosine kinase, a pharmaceutical composition and a method for preparing the compound.

The problem is solved by the features of claims 1, 7, 9 and 10. Embodiments are subject-matter of claims 2 to 6, 8 and 11 to 15.

According to the invention a compound is provided, wherein the compound is a substance according to the following formula: 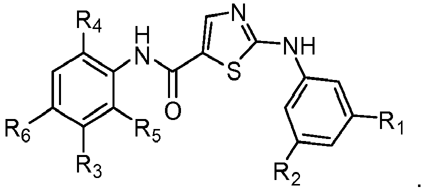
R₁ is 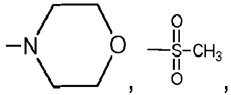 -SO₃H, 1-methyl-4,5-dihydro-1H-imidazol-2-yl, 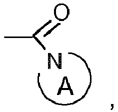 or 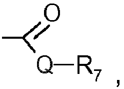 wherein the ring A is a pyrrolidine, piperidine, morpholine, or imidazole residue or a substituted pyrrolidine, piperidine, morpholine, or imidazole residue, Q is -NH-, -NH-alkyl-, -O-, or -S- and R₇ is -H, a substituted or non-substituted pyrrolidine, piperidine, morpholine, triazine, or imidazole residue, or a substituted or non-substituted alkylpyrrolidine, alkylpiperidine, alkylmorpholine, alkyltriazine, or alkylimidazole residue, alkyl, alkylester, alkenyl, alkenylester, alkynyl, alkynylester, aminoalkyl, cyanyl, -NO₂, methylsulfonyl residue, or a substituted or non-substituted form of any of the following residues: cycloalkyl, cycloalkylalkyl, cycloalkenyl, cycloalkenylalkyl, bicycloalkyl, bicycloalkylalkyl, bicycloalkenyl, bicycloalkenylalkyl, or another bicyclic residue, aryl, aminoaryl, heteroaryl, heteroaminoaryl, cyclyl, aminocyclyl, heterocyclyl, heteroaminocyclyl, aralkyl, aminoaralkyl, heterocycloalkyl, or heteroaminocycloalkyl.
R₂ is 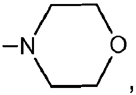 -H, -Cl, -Br, -CF₃, 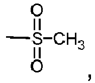 -SO₃H, -CH=CH₂ or another alkenyl, -C≡CH or another alkynyl, phenyl or substituted phenyl, triazolyl or another heteroaryl, substituted triazolyl or another substituted heteroaryl, a residue of 9-BBN (9-borabicyclo[3.3.1]nonane), OBBD (9-oxa-10-borabicyclo[3.3.2]decane), trifluoroborate, boronic acid, or an ester of boronic acid and pinacol, methyliminodiacetic acid (MIDA), catechol, pinane diol, ethylene glycol, or propane diol or another boronic acid ester, 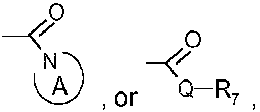 wherein the ring A is a pyrrolidine, piperidine, morpholine, or imidazole residue or a substituted pyrrolidine, piperidine, morpholine, or imidazole residue, Q is -NH-, -NH-alkyl-, -O-, or -S- and R₇ is -H, a substituted or non-substituted pyrrolidine, piperidine, morpholine, triazine, or imidazole residue, or a substituted or non-substituted alkylpyrrolidine, alkylpiperidine, alkylmorpholine, alkyltriazine, or alkylimidazole residue, alkyl, alkylester, alkenyl, alkenylester, alkynyl, alkynylester, aminoalkyl, cyanyl, -NO₂, methylsulfonyl residue, or a substituted or non-substituted form of any of the following residues: cycloalkyl, cycloalkylalkyl, cycloalkenyl, cycloalkenylalkyl, bicycloalkyl, bicycloalkylalkyl, bicycloalkenyl, bicycloalkenylalkyl, or another bicyclic residue, aryl, aminoaryl, heteroaryl, heteroaminoaryl, cyclyl, aminocyclyl, heterocyclyl, heteroaminocyclyl, aralkyl, aminoaralkyl, heterocycloalkyl, or heteroaminocycloalkyl.
R₃ is -H or -CH₃.
R₄ is -H, -Cl, -Br, -CH₃, -CH(CH₃)₂ or -C(CH₃)₃.
R₅ is -H, -CH₃, -CH(CH₃)₂ or -C(CH₃)₃.
R₆ is -H or -CH₃.

In any case the compound may also be a salt of said substance.

The structure according to formula (I) is similar to the structure of dasatinib. However, the structure clearly deviates from the structure of dasatinib in residues R₁ and R₂ and in the absence of a pyrimidine ring. The compound according to the invention also differs from the compounds known from WO 2005/000298 A2 and WO 2008/150446 A1.

The inventors found that the substance according to formula (I) or a salt of said substance can be used as inhibitor of activity of a protein tyrosine kinase of a human being or an animal, in particular a mammal. For example, the compound according to the invention can be used in a first line treatment of a cancer. The compound according to the invention can also be used in an alternative or additional treatment of a cancer treated with an antibody if a resistance vis-à-vis this antibody occurs. The antibody may be Trastuzumab. The compound according to the invention can also be used in an alternative or additional treatment of a cancer treated with a protein tyrosine kinase inhibitor such as imatinib, nilotinib, gefitinib, dasatinib, or vemurafenib, if a resistance vis-à-vis this protein tyrosine kinase inhibitor occurs.

The inventors found that compounds according to the invention have a relatively high affinity to the protein tyrosine kinase(s) ephrin receptor EphA2 and/or ABL1 and/or ABL2 and a high selectivity with respect to this/these tyrosine kinase(s). Furthermore, experimental data using the glioblastoma cell line SF-268 showed that cells of this cell line have a high expression of EphA2 and a relatively low expression of other protein targets of dasatinib, such as kinases. Cells of cell line SF-268 are available from the Developmental Therapeutics Program DTP at the National Cancer Institute NCI. Inhibition of EphA2 expression resulted in a reduced viability of these cells indicating that viability of these cells depend on expression and probably activity of EphA2. Treatment of these cells with compounds according to the invention resulted in a clearly reduced cell viability.

The invention also encompasses a prodrug that is metabolized in vivo to a substance according to formula (I) or a salt/ion of said substance. A prodrug can be generated by modification of any of residues R₁, R₂, R₃, R₄, R₅, R₆, and R₇, in particular by modification of residue R₁ or R₇. The modification may be the introduction of an ester. An example of a prodrug is the compound according to the invention, wherein R₇ is an alkylester, alkenylester, or alkynylester. The residue bound via the ester, such as alkyl in case of alkylester, may provide enhanced hydrophobicity resulting in increased cell permeability. Upon cell entry, intracellular esterases may cleave the ester and release another compound according to the invention.

In an embodiment of the invention R₁ is 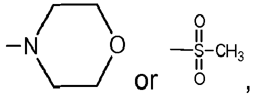 R₂ is 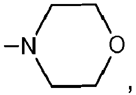 R₃ is -H, R₄ is -Cl and R₅ is -CH₃, -CH(CH₃)₂ or -C(CH₃)₃.

In an another embodiment of the invention R₁ is 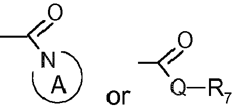 , R₂ is -H or -Br, R₃ is -H or -CH₃, R₄ is -H, -Cl, -CH₃, -CH(CH₃)₂ or -C(CH₃)₃ and R₅ is -H, -CH₃, -CH(CH₃)₂ or -C(CH₃)₃ and R₇ is a substituted or non-substituted pyrrolidine, piperidine, triazine, or imidazole residue, or a substituted or non-substituted alkylpyrrolidine, alkylpiperidine, alkyltriazine, or alkylimidazole.

In a further embodiment of the invention R₁ is 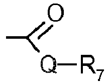 and R₂ is -H, -Br, -CF₃, -CH=CH₂, phenyl or substituted phenyl. R₇ may be a substituted or non-substituted pyrrolidine, piperidine, triazine, or imidazole residue, or a substituted or non-substituted alkylpyrrolidine, alkylpiperidine, alkyltriazine, or alkylimidazole.

In another embodiment of the invention R₁ is 1-methyl-4,5-dihydro-1H-imidazol-2-yl, R₂ is -H or -Br, R₃ is -H, R₄ is -Cl and R₅ is -CH₃, -CH(CH₃)₂ or -C(CH₃)₃.

The substance may be any of the substances characterized according to the following tables:

| **#** | **structure** | **analytical data** |
|---|---|---|
| **1** | 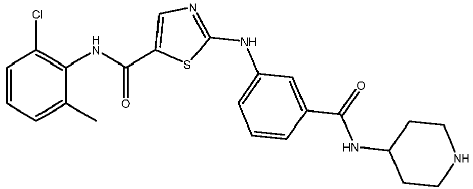 | ¹H NMR (400 MHz, DMSO-d6) δ 9.95 (s, 1H), 8.26 (d, J = 7.8 Hz, 1H), 8.15 (s, 1H), 8.03 (s, 1H), 7.84 (d, J = 7.8 1H), 7.43 (m, 3H), 7.28 (m, 2H), 3.83 (m, 1H), 2.98 (m, 2H), 2.55 (m, 2H), 2.23 (s, 3H), 1.74 (m, 2H), 1.44 (m, 2H); ¹³C NMR (101 MHz, DMSO) δ 166.58, 165.41, 159.17, 142.32, 140.33, 138.76, 135.94, 133.39, 132.37, 129.03, 128.83, 128.20, 127.00, 122.77, 120.67, 120.02, 117.04, 47.26, 45.10, 32.58, 18.27; m/z [M+H]+ : 470.1 |
| | N-(2-Chloro-6-methylphenyl)-2-((3-(piperidin-4-ylcarbamoyl)phenyl)amino)thiazole-5-carboxamide | |
| **2** | 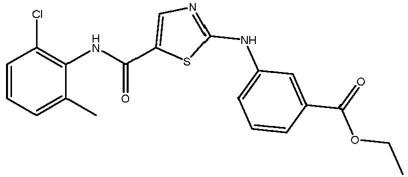 | ¹H NMR (400 MHz, DMSO-d6) δ 10.85 (s, 1H), 9.95 (s, 1H), 8.29 (s, 1H), 8.16 (s, 1H), 7.94 (m, 2H), 7.60 (d, J = 7.8 Hz, 1H), 7.50 (t, J = 7.9 Hz, 1H), 7.40 (dd, J = 7.5, 1.7 Hz, 2H), 7.28 (m, 3H), 4.33 (q, J = 7.1 Hz, 2H), 2.24 (s, 3H), 1.33 (t, J = 7.1 Hz, 3H); ¹³C NMR (101 MHz, DMSO-d6) δ 166.33, 165.58, 159.11, 142.24, 140.65, 138.76, 133.34, 132.36, 130.68, 129.50, 129.04, 128.23, 127.01, 123.12, 122.57, 121.92, 117.90, 60.82, 18.26, 14.15; m/z [M+H]+ : 416.0 |
| | Ethyl 3-((5-((2-chloro-6-methylphenyl)carbamoyl)thiazol-2-yl)amino)benzoate | |
| **3** | 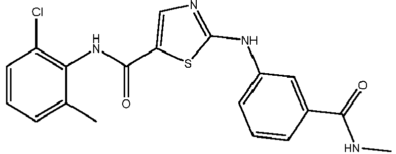 | ¹H NMR (400 MHz, DMSO-d6) δ 10.75 (s, 1H), 9.94 (s, 1H), 8.41 (q, J = 4.3 Hz, 1H), 8.15 (s, 1H), 8.08 (s, 1H), 7.79 (d, J = 7.1 Hz, 1H), 7.41 (m, 3H), 7.28 (m, 2H), 2.78 (d, J sq= 4.5 Hz, 3H), 2.23 (s, 3H); ¹³C NMR (101 MHz, DMSO-d6) δ 170.20, 166.58, 159.17, 142.33, 140.42, 138.77, 135.65, 133.37, 132.37, 129.04, 129.00, 128.23, 127.01, 120.39, 120.35, 120.08, 116.76, 26.29, 18.28; m/z [M+H]+ : 401.0 |
| | N-(2-Chloro-6-methylphenyl)-2-((3-(methylcarbamoyl)phenyl)amino)thiazole-5-carboxamide | |
| **4** | 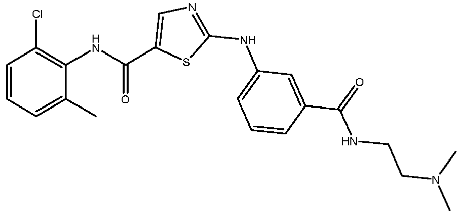 | ¹H NMR (400 MHz, Methanol-d4) δ 8.12 (s, 1H), 8.07 (s, 1H), 7.75 (d, J = 7.8 1H), 7.50 (d, J = 7.81H), 7_{.}44 (t, J = 7.8 Hz, 1H), 7.35 (dd, J = 7.2, 2.0 Hz, 1H), 7.24 (m, 2H), 3.56 (t, J = 6.8 Hz, 2H), 2.65 (t, J = 6.8 Hz, 2H), 2.37 (s, 6H), 2.32 (s, 3H), 2.10 (s, 1H); ¹³C NMR (101 MHz, Methanol-d4) δ 170.11, 169.50, 162.44, 143.83, 141.96, 140.35, 136.82, 134.28, 134.24, 130.38, 130.15, 130.09, 129.60, 128.34, 122.48, 122.45, 118.45, 59.17, 45.44, 38.49, 18.68; m/z [M+H]+ : 458.1 |
| | N-(2-Chloro-6-methylphenyl)-2-((3-((2-(dimethylamino)ethyl)carbamoyl)phenyl)amino) thiazole-5-carboxamide | |
| **5** | 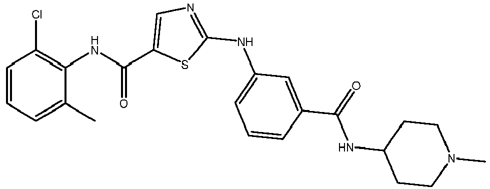 | ¹H NMR (400 MHz, Methanol-d4) δ 8.07 (m, 2H), 7.73 (dd, J = 7.8, 2.2 Hz, 1H), 7.46 (m, 2H), 7.35 (dd, J = 7.4, 2.1 Hz, 1H), 7.25 (m, 2H), 3.92 (m, 1H), 2.96 (m, 2H), 2.34 (m, 8H), 1.98 (m, 2H), 1.74 (m, 2H); ¹³C NMR (101 MHz, Methanol-d4) δ 169.83, 169.56, 162.42, 143.75, 141.83, 140.35, 137.15, 134.27, 134.24, 130.34, 130.15, 129.60, 128.34, 123.77, 122.63, 122.44, 118.54, 55.56, 45.97, 45.90, 32.06, 18.68; m/z [M+H]+ : 484.1 |
| | N-(2-Chloro-6-methylphenyl)-2-((3-((1-methylpiperidin-4-yl)carbamoyl)phenyl)amino) thiazole-5-carboxamide | |
| **6** | 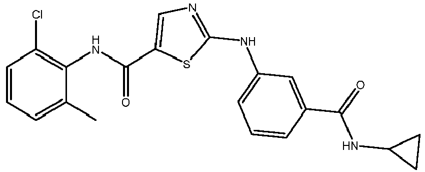 | ¹H NMR (400 MHz, DMSO-d6) δ 10.75 (s, 1H), 9.95 (s, 1H), 8.44 (d, J = 4.2 Hz, 1H), 8.14 (s, 1H), 8.04 (s, 1H), 7.80 (d, J = 6.9 Hz, 1H), 7.40 (m, 3H), 7.27 (m, 2H), 2.85 (m, 1H), 2.23 (s, 3H), 0.69 (m, 2H), 0.57 (m, 2H); ¹³C NMR (101 MHz, DMSO-d6) δ 167.52, 166.61, 159.22, 142.35, 140.40, 138.81, 135.60, 133.40, 132.40, 129.08, 128.94, 128.27, 127.05, 122.84, 120.60, 120.18, 116.87, 23.10, 18.30, 5.75; m/z [M+H]+ : 426.1 |
| | N-(2-Chloro-6-methylphenyl)-2-((3-(cyclopropylcarbamoyl)phenyl)amino)thiazole-5-carboxamide | |
| **7** | 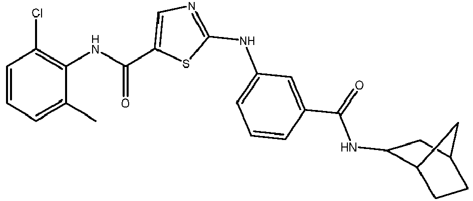 | ¹H NMR (400 MHz, Methanol-d4) δ 8.06 (s, 1H), 8.03 (s, 1H), 7.70 (d, J = 7.4, 2.1 Hz, 1H), 7.43 (m, 2H), 7.35 (dd, J = 7.2, 2.2 Hz, 1H), 7.23 (m, 2H), 3.81 (m, 1H), 2.31 (s, 3H), 1.79 (m, 1H), 1.56 (m, 5H), 1.25 (m, 4H); ¹³C NMR (101 MHz, Methanol-d4) δ 169.95, 169.64, 162.43, 143.72, 141.73, 140.35, 137.48, 134.28, 134.26, 130.31, 130.14, 129.58, 128.33, 123.76, 122.74, 122.30, 118.56, 55.19, 43.63, 39.86, 37.06, 36.23, 29.36, 27.61, 18.67; m/z [M+H]+ : 481.1 |
| | 2-((3-((1S,4R)-Bicyclo[2.2.1]heptan-2-ylcarbamoyl)phenyl)amino)-N-(2-chloro-6-methylphenyl)thiazole-5-carboxamide | |
| **8** | 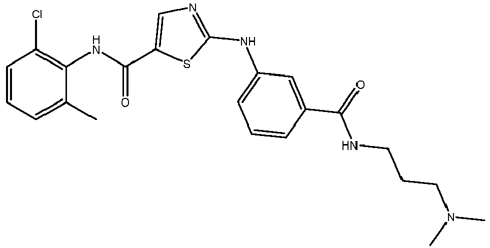 | ¹H NMR (400 MHz, Methanol-d4) δ 8.10 (s, 1H), 8.07 (s, 1H), 7.74 (d, J = 7.2 Hz, 1H), 7.45 (m, 2H), 7.35 (dd, J = 7.0, 2.1 Hz, 1H), 7.24 (m, 2H), 3.43 (t, J = 7.0 Hz, 2H), 2.46 (t, J = 7.0 2H), 2.32 (s, 3H), 2.30 (s, 6H), 1.83 (q, J = 7.0 2H); ¹³C NMR (101 MHz, Methanol-d4) δ 170.12, 169.56, 162.45, 143.81, 141.98, 140.35, 137.07, 134.29, 134.26, 130.40, 130.18, 130.15, 129.59, 128.35, 122.42, 122.41, 118.39, 58.33, 45.40, 39.38, 28.04, 18.66; m/z [M+H]+ : 472.1 |
| | N-(2-Chloro-6-methylphenyl)-2-((3-((3-(dimethylamino)propyl)carbamoyl)phenyl)amino) thiazole-5-carboxamide | |
| **9** | 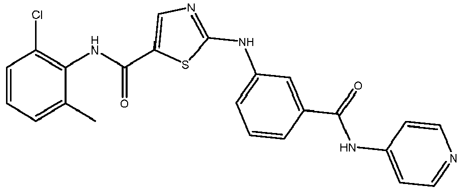 | m/z [M+H]+ : 464.1 |
| | N-(2-Chloro-6-methylphenyl)-2-((3-(pyridin-4-ylcarbamoyl)phenyl)amino)thiazole-5-carboxamide | |
| **10** | 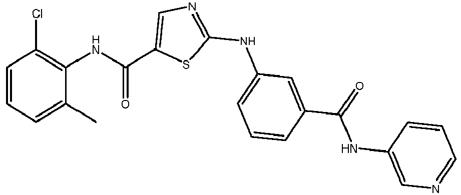 | ¹H NMR (500 MHz, DMSO-d6) δ 10.84 (s, 1H), 10.48 (s, 1H), 9.95 (s, 1H), 8.93 (d, J = 2.5 Hz, 1H), 8.32 (dd, J = 4.7, 1.4 Hz, 1H), 8.19 (m, 3H), 7.89 (dd, J = 8.2, 2.4 Hz, 1H), 7.62 (d, J = 7.7 Hz, 1H), 7.53 (t, J = 7.9 Hz, 1H), 7.40 (dd, J = 8.4, 4.7 Hz, 2H), 7.27 (m, 2H), 2.24 (s, 3H); ¹³C NMR (126 MHz, DMSO-d6) δ 166.45, 165.92, 159.12, 144.58, 142.27, 141.95, 140.57, 138.75, 135.76, 135.43, 133.34, 132.35, 129.17, 129.02, 128.21, 127.28, 126.99, 123.50, 123.02, 121.02, 120.77, 117.01, 18.25; m/z [M+H]+ : 464.1 |
| | N-(2-Chloro-6-methylphenyl)-2-((3-(pyridin-3-ylcarbamoyl)phenyl)amino)thiazole-5-carboxamide | |
| **11** | 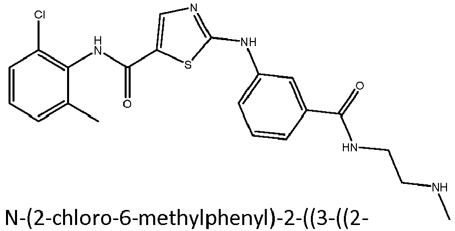 | m/z [M+H]+ : 444.0 |
| | (methylamino)ethyl)carbamoyl)phenyl)amino)thiazole-5-carboxamide | |
| **12** | 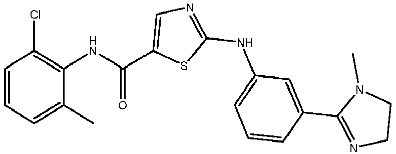 | m/z [M+H]+ : 426.0 |
| | N-(2-chloro-6-methylphenyl)-2-((3-(1-methyl-4,5-dihydro-1H-imidazol-2-yl)phenyl)amino)thiazole-5-carboxamide | |
| **13** | 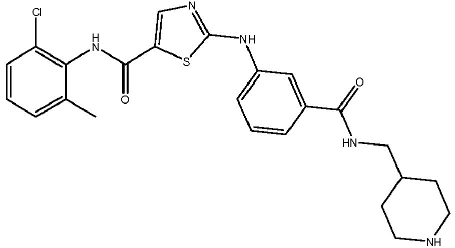 | ¹H NMR (400 MHz, DMSO-d6) δ 9.95 (s, 1H), 8.44 (t, *J* = 5.8 Hz, 1H), 8.15 (s, 1H), 8.06 (s, 1H), 7.82 (d, *J* = 7.8 Hz, 1H), 7.52 - 7.34 (m, 3H), 7.34-7.17 (m, 2H), 3.35 (s, 2H), 3.12 (t, *J* = 6.2 Hz, 2H), 2.94 (d, *J* = 12.0 Hz, 2H), 2.60 (s, 1H), 2.43 (t, *J* = 12.0 Hz, 2H), 2.24 (s, 3H), 1.61 (d, *J* = 12.0 Hz, 2H), 1.05 (dd, *J* = 12.0, 3.8 Hz, 2H); ¹³C NMR (101 MHz, DMSO-d6) δ 167.05, 166.75, 159.66, 142.80, 140.89, 139.26, 136.37, 133.88, 132.87, 129.53, 129.40, 128.71, 127.50, 123.28, 120.98, 120.51, 117.38, 47.42, 46.17, 36.71, 31.22, 18.76; m/z [M+H]+ : 484.1 |
| | N-(2-Chloro-6-methylphenyl)-2-((3-((piperidin-4-ylmethyl)carbamoyl)phenyl)amino) thiazole-5-carboxamide | |
| **14** | 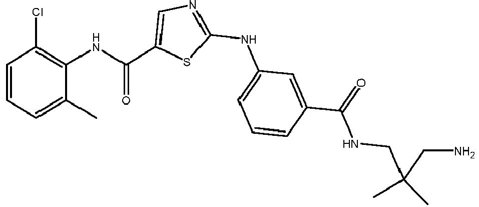 | ¹H NMR (500 MHz, Methanol-d4) δ 8.08 (s, 1H), 8.01 (s, 1H), 7.66 (d, J = 7.7 Hz, 1H), 7.40 (dt, J = 15.5, 7.7 Hz, 2H), 7.28 (dd, J = 9.1, 7.7 Hz, 1H), 7.21-7.13 (m, 2H), 3.23 (s, 2H), 2.46 (s, 2H), 2.25 (s, 3H), 0.94 (s, 6H); ¹³C NMR (126 MHz, Methanol-*d*₄) δ 171.12, 169.64, 162.57, 143.90, 142.10, 140.48, 137.08, 134.39, 130.58, 130.29, 129.75, 128.49, 123.96, 122.62, 122.61, 118.56, 50.17, 47.91, 37.53, 30.93, 24.11, 18.83; m/z [M+H]+ : 472.2 |
| | 2-((3-((3-amino-2,2-dimethylpropyl)carbamoyl)phenyl)amino)-N-(2-chloro-6-methylphenyl)thiazole-5-carboxamide | |
| **15** | 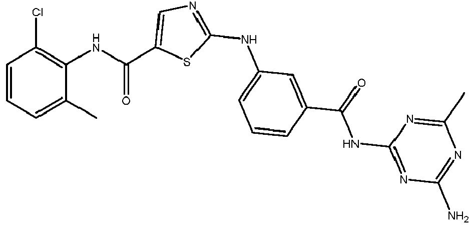 | ¹H NMR (400 MHz, DMSO-d6) δ 9.97 (s, 1H), 8.17-8.10 (m, 2H), 7.55 (d, *J* = 8.1 Hz, 1H), 7.26 (d, *J* = 7.5 Hz, 2H), 2.25 (s, 3H), 2.23 (s, 3H), 7.37 - 7.31 (m, 1H), 10.82 (s, 1H), 10.62 (s, 1H), 7.86 (dd, *J* = 7.9, 2.6 Hz, 1H), 7.45 (t, *J* = 7.9 Hz, 1H), 10.03 - 9.90 (m, 1H), 7.39 (dd, J = 7.5, 2.0 Hz, 1H); ¹³C NMR (101 MHz, DMSO-d6) δ 176.26, 167.38, 166.61, 165.77, 164.40, 159.33, 142.40, 140.48, 138.89, 135.32, 133.45, 132.48, 129.23, 129.18, 128.40, 127.14, 123.07, 121.88, 121.24, 117.56, 24.93, 18.38; m/z [M+H]+ : 495.1 |
| | 2-((3-((4-amino-6-methyl-1,3,5-triazin-2-yl)carbamoyl)phenyl)amino)-N-(2-chloro-6-methylphenyl)thiazole-5-carboxamide | |
| **16** | 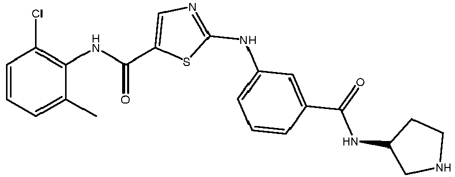 | ¹H NMR (500 MHz, Methanol-*d*₄) δ 8.06 (s, 1H), 7.93 (d, *J* = 8.6 Hz, 1H), 7.68 - 7.59 (m, 1H), 7.44 (t, *J* = 7.9 Hz, 1H), 7.35 (dd, *J* = 7.6, 2.0 Hz, 1H), 7.23 (ddt, *J* = 12.6, 9.1, 6.6 Hz, 3H), 3.86 - 3.75 (m, 1H), 3.75 - 3.61 (m, 2H), 3.61 - 3.39 (m, 2H), 2.31 (s, 3H), 2.28 - 2.10 (m, 1H), 1.84 (ddt, *J* = 18.4, 12.3, 6.3 Hz, 1H).; ¹³C NMR (101 MHz, Methanol-*d*₄) δ 162.47, 143.93, 141.91, 140.34, 138.70, 134.27, 134.24, 130.42, 130.14, 129.60, 128.34, 123.74, 122.27, 121.02, 120.94, 117.90, 117.83, 45.81, 34.67, 32.99, 18.68, 17.10; m/z [M+H]+ : 456.2 |
| | (R)-N-(2-chloro-6-methylphenyl)-2-((3-(pyrrolidin-3-ylcarbamoyl)phenyl)amino)thiazole-5-carboxamide | |
| **17** | 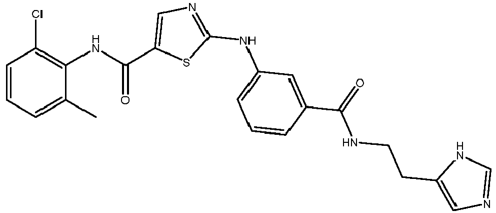 | m/z [M+H]+ : 481.2 |
| | 2-((3-((2-(1H-imidazol-5-yl)ethyl)carbamoyl)phenyl)amino)-N-(2-chloro-6-methylphenyl)thiazole-5-carboxamide | |
| **18** | 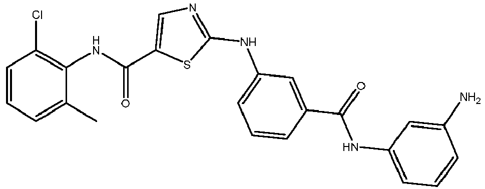 | ¹H NMR (500 MHz, Methanol-d4) δ 8.17 (s, 1H), 8.08 (s, 1H), 7.76 (d, J = 7.3 Hz, 1H), 7.55 (dd, J = 15.0, 5.4 Hz, 1H), 7.48 (t, J = 7.9 Hz, 1H), 7.34 (dt, J = 11.2, 5.5 Hz, 1H), 7.29 - 7.19 (m, 2H), 7.16 (d, J = 1.7 Hz, 1H), 7.09 (t, J = 8.0 Hz, 1H), 7.00 - 6.94 (m, 1H), 6.57 - 6.51 (m, 1H), 2.32 (s, 3H); ¹³C NMR (101 MHz, Methanol-*d*₄) δ 169.52, 168.74, 162.44, 149.40, 143.76, 141.89, 140.52, 140.35, 137.85, 134.26, 134.25, 130.45, 130.34, 130.15, 129.61, 128.35, 123.84, 122.84, 122.51, 118.58, 113.09, 112.15, 109.38, 18.68; m/z [M+H]+ : 478.1 |
| | 2-((3-((3-aminophenyl)carbamoyl)phenyl)amino)-N-(2-chloro-6-methylphenyl)thiazole-5-carboxamide | |
| **19** | 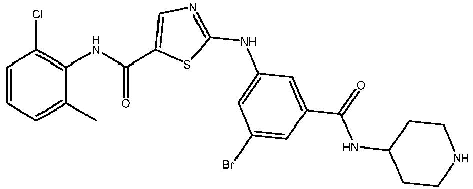 | ¹H NMR (400 MHz, methanol-d4) δ 8.09 (s, 1H), 8.00 (t, J = 1.7 Hz, 1H), 7.74 (t, J = 1.4 Hz, 1H), 7.36 (dd, J = 7.2, 2.0 Hz, 1H), 7.25 (m, 2H), 7.21 (t, J = 1.5 Hz, 1H), 4.58 (d, J = 7.0 Hz, 1H), 3.77 (d, J = 10.1 Hz, 1H), 3.19 (t, J = 10.0 Hz, 1H), 3.01 (m, 1H), 2.96 (t, J = 10.0 Hz, 1H), 2.32 (s, 3H), 2.00 (d, J = 8.0 Hz, 1H), 1.88 (d, J = 8.0 Hz, 1H), 1.42 (t, J = 9.8 Hz, 2H); m/z [M+H]+ : 548.0 |
| | 2-((3-bromo-5-(piperidin-4-ylcarbamoyl)phenyl)amino)-N-(2-chloro-6-methylphenyl)thiazole-5-carboxamide | |
| **20** | 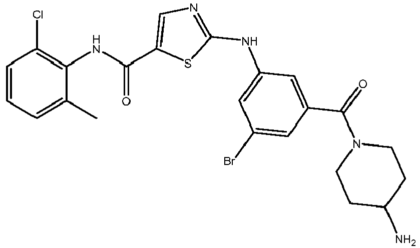 | ¹H NMR (400 MHz, Methanol-*d*₄) δ 8.16 (t, *J* = 1.9 Hz, 1H), 8.11 (s, 1H), 8.01 (t, *J* = 1.8 Hz, 1H), 7.64 (t, *J* = 1.6 Hz, 1H), 7.37 (dd, *J* = 7.2, 2.3 Hz, 1H), 7.29-7.22 (m, 2H), 3.49 (dt, *J* = 13.1, 3.7 Hz, 2H), 3.17 (td, *J* = 12.8, 3.1 Hz, 2H), 2.33 (s, 3H), 2.26-2.19 (m, 2H), 1.94-1.82 (m, 2H); m/z [M+H]+ : 458.0 |
| | 2-((3-(4-aminopiperidine-1-carbonyl)-5-bromophenyl)amino)-N-(2-chloro-6-methylphenyl)thiazole-5-carboxamide | |
| **21** | 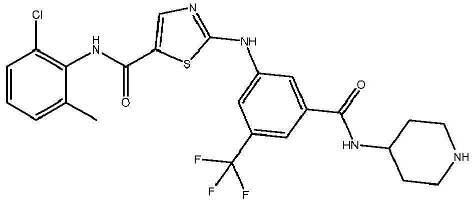 | ¹H NMR (400 MHz, methanol-d4) δ 8.11 (s, 2H), 8.06 (s, 1H), 7.33 (m, 2H), 7.25 (m, 2H), 4.63 (m, 1H), 3.77 (m, 1H), 3.23 (m, 1H), 3.11 (tt, J = 11.0, 3.9 Hz, 1H), 2.99 (m, 1H), 2.32 (s, 3H), 2.03 (m, 1H), 1.91 (m, 1H), 1.46 (m, 2H); ¹³C NMR (101 MHz, Methanol-*d*₄) δ 170.54, 168.34, 164.87, 162.29, 143.76, 142.92, 140.32, 138.95, 134.21, 133.15, 130.18, 129.67, 128.37, 124.97, 120.08, 117.77, 116.47, 47.63, 42.12, 36.94, 34.65, 33.85, 31.64, 18.66; m/z [M+H]+ : 538.2 |
| | N-(2-chloro-6-methylphenyl)-2-((3-(piperidin-4-ylcarbamoyl)-5-(trifluoromethyl)phenyl)amino)thiazole-5-carboxamide | |
| **22** | 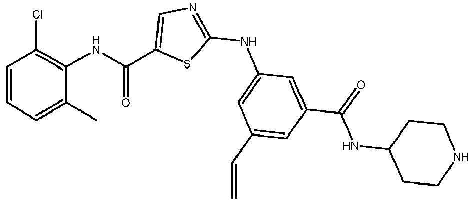 | ¹H NMR (400 MHz, methanol-d4) δ 8.07 (s, 1H), 7.73 (t, J = 1.7 Hz, 1H), 7.70 (t, J = 1.7 Hz, 1H), 7.36 (dd, J = 7.2, 2.0 Hz, 1H), 7.25 (m, 2H), 7.16 (t, J = 1.2 Hz, 1H), 6.78 (dd, J = 18.0, 10.9 Hz, 1H), 5.87 (d, J = 17.7 Hz, 1H), 5.36 (d, J = 11.1 Hz, 1H), 4.63 (d, J = 9.8 Hz, 1H), 3.85 (d, J = 9.0 Hz, 1H), 3.21 (t, J = 13.7 Hz, 1H), 3.12 (tt, J = 11.0, 4.1 Hz, 1H), 2.96 (t, J = 11.7 Hz, 1H), 2.32 (s, 3H), 2.03 (m, 1H), 1.91 (m, 1H), 1.47 (t, J = 8.8 Hz, 2H); m/z [M+H]+ : 496.2 |
| | N-(2-chloro-6-methylphenyl)-2-((3-(piperidin-4-ylcarbamoyl)-5-vinylphenyl)amino)thiazole-5-carboxamide | |
| **23** | 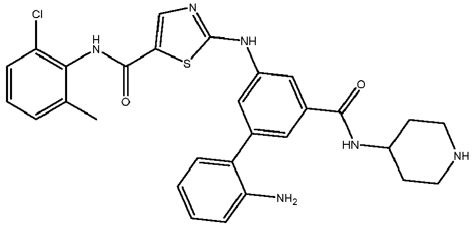 | ¹H NMR (400 MHz, methanol-d4) δ 8.07 (s, 1H), 7.85 (s, 1H), 7.67 (s, 1H), 7.35 (d, J = 7.1 Hz, 1H), 7.25 (m, 2H), 7.16 (s, 1H), 7.12 (m, 2H), 6.86 (d, J = 8.0 Hz, 1H), 6.79 (t, J = 7.5 Hz, 1H), 4.74 (m, 1H), 4.04 (m, 1H), 3.45 (tt, J = 11.5, 3.7 Hz, 1H), 3.29 (m, 1H), 2.99 (m, 1H), 2.31 (s, 3H), 2.13 (m, 1H), 2.05 (m, 1H), 1.63 (m, 2H); ¹³C NMR (101 MHz, Methanol-*d*₄) δ 172.20, 169.23, 164.87, 162.42, 145.39, 143.98, 143.09, 142.54, 140.33, 137.80, 134.22, 131.16, 130.17, 130.07, 129.66, 128.36, 127.63, 123.84, 122.50, 121.49, 119.62, 117.46, 115.94, 47.08, 41.51, 36.95, 31.65, 30.75, 18.68; m/z [M+H]+ : 561.2 |
| | 2-((2'-amino-5-(piperidin-4-ylcarbamoyl)-[1,1'-biphenyl]-3-yl)amino)-N-(2-chloro-6-methylphenyl)thiazole-5-carboxamide | |
| **24** | 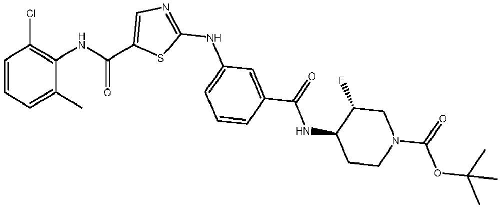 | ¹H NMR (360 MHz, Methanol-*d*₄) δ 8.10 (s, 1H), 8.07 (s, 1H), 7.75 (d, *J* = 8.0 Hz, 1H), 7.52 - 7.41 (m, 2H), 7.35 (dd, *J* = 7.1, 2.3 Hz, 1H), 7.28-7.18 (m, 2H), 4.90 (s, 1H), 4.55 (d, *J* = 193.8 Hz, 1H), 4.42 (tdd, *J* = 9.0, 4.7, 0.0 Hz, 1H), 4.34 - 4.15 (m, 2H), 3.98 (d, *J* = 14.0 Hz, 1H), 2.32 (s, 3H), 2.04 (dd, J = 11.9, 7.3 Hz, 1H), 1.69 - 1.56 (m, 1H), 1.48 (d, *J* = 1.3 Hz, 9H).; m/z [M+H]+ : 488.1 (Boc deprotection in acidic LC conditions) |
| | tert-butyl (3R,4R)-4-(3-((5-((2-chloro-6-methylphenyl)carbamoyl)thiazol-2-yl)amino)benzamido)-3-fluoropiperidine-1-carboxylate | |
| **25** | 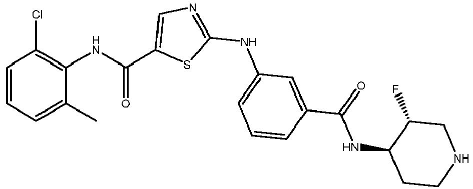 | ¹H NMR (360 MHz, Methanol-*d*₄) δ 8.13 (t, *J* = 1.9 Hz, 1H), 8.08 (s, 1H), 7.72 (dd, *J* = 7.8, 1.6 Hz, 1H), 7.53 (td, 1H), 7.46 (t, *J* = 7.9 Hz, 1H), 7.35 (dd, *J* = 7.2, 2.3 Hz, 1H), 7.28-7.18 (m, 2H), 5.12 - 5.01 (m, 1H), 4.49-4.35 (m, 1H), 3.65 (ddd, *J* = 20.8, 13.3, 3.4 Hz, 1H), 3.52 - 3.35 (m, 2H), 3.30 - 3.20 (m, 1H), 2.42 - 2.33 (m, 1H), 2.31 (s, 3H), 2.09-1.91 (m, 1H); ¹³C NMR (91 MHz, Methanol-*d₄*) δ 170.56, 169.58, 160.65, 143.34, 141.78, 140.32, 136.54, 134.20, 130.48, 130.15, 129.62, 128.33, 123.03, 122.94, 118.80, 87.40, 85.43, 45.49, 45.20, 42.22, 25.47, 25.43, 18.6; m/z [M+H]+ : 488.1 |
| | N-(2-chloro-6-methylphenyl)-2-((3-(((3R,4R)-3-fluoropiperidin-4-yl)carbamoyl)phenyl)amino)thiazole-5-carboxamide | |
| **26** | 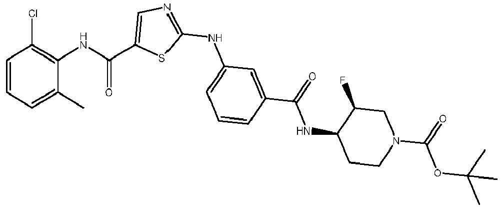 | m/z [M+H]+ : 488.1 (Boc deprotection in acidic LC conditions) |
| | tert-butyl (3S,4R)-4-(3-((5-((2-chloro-6-methylphenyl)carbamoyl)thiazol-2-yl)amino)benzamido)-3-fluoropiperidine-1-carboxylate | |
| **27** | 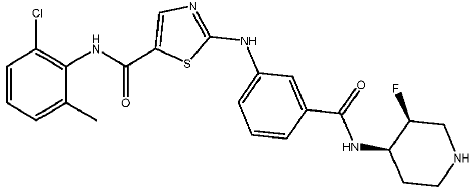 | ¹H NMR (360 MHz, Methanol-d4) δ 8.20 - 8.12 (m, 1H), 8.09 (s, 1H), 7.81 - 7.69 (m, 1H), 7.58 - 7.46 (m, 2H), 7.38 (dd, J = 7.1, 2.5 Hz, 1H), 7.32 - 7.23 (m, 2H), 5.18 (d, J = 47.6 Hz, 1H), 4.44 (dddd, J = 31.1, 12.5, 4.8, 2.0 Hz, 1H), 3.77 (ddt, J = 11.8, 9.6, 2.6 Hz, 1H), 3.63 - 3.39 (m, 2H), 3.27 (dd, J = 13.2, 3.3 Hz, 1H), 2.35 (s, 3H), 2.28 (dd, J = 13.2, 4.4 Hz, 1H), 2.12 (d, J = 13.8 Hz, 1H); m/z [M+H]+ : 488.1 |
| | N-(2-chloro-6-methylphenyl)-2-((3-(((3S,4R)-3-fluoropiperidin-4-yl)carbamoyl)phenyl)amino)thiazole-5-carboxamide | |
| **28** | 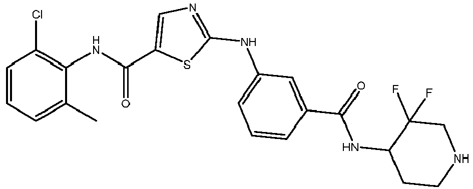 | ¹H NMR (500 MHz, DMSO-d6) δ 10.83 (s, 1H), 9.98 (s, 1H), 8.15 (s, 1H), 7.79 (s, 1H), 7.66 (d, *J* = 8.1 Hz, 1H), 7.51 - 7.34 (m, 2H), 7.33-7.21 (m, 2H), 2.01 - 1.68 (m, 2H), 7.10 - 6.94 (m, 1H), 3.54 (d, *J* = 36.0 Hz, 1H), 3.31 - 3.05 (m, 2H), 2.23 (s, 3H), 2.08 - 1.67 (m, 2H), 1.51 (d, *J* = 11.5 Hz, 1H), 1.23 (s, 1H); m/z [M+H]+ : 506.1 |
| | N-(2-chloro-6-methylphenyl)-2-((3-((3,3-difluoropiperidin-4-yl)carbamoyl)phenyl)amino)thiazole-5-carboxamide | |
| **29** | 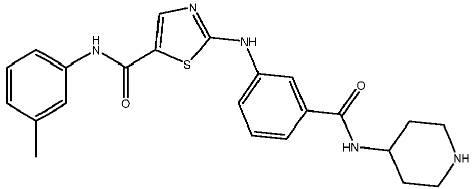 | 1H-NMR(360 MHz, Methanol-d4) δ 7.98 (s, 1H), 7.79 (t ,J = 1.9 Hz, 1H), 7.56 (ddd, J = 8.3, 2.4, 1.0 Hz, 1H), 7.41 (d, J = 2.0 Hz, 1H), 7.37 (d, J = 8.0 Hz, 2H), 7.17 (t, J = 7.8 Hz, 1H), 7.03 (dt, J = 7.5, 1.3 Hz, 1H), 6.91 (d, J = 7.6 Hz, 1H), 4.55 (d, J = 13.5 Hz, 1H), 3.76 (d, J = 13.7 Hz, 1H), 3.19 - 3.03 (m, 1H), 2.92 (s, 1H), 2.30 (s, 3H), 1.91 (d, J = 10.5 Hz, 1H), 1.80 (s, 1H), 1.35 (s, 3H); 13C-NMR(91 MHz, Methanol-d4) δ 172.09, 169.11, 164.85, 161.87, 143.18, 142.04, 139.78, 139.45, 137.97, 130.58, 129.65, 126.18, 125.22, 122.64, 121.84, 120.59, 119.20, 117.43, 36.94, 31.65, 21.56; m/z [M+H]+ : 436.1 |
| | 2-((3-(piperidin-4-ylcarbamoyl)phenyl)amino)-N-(m-tolyl)thiazole-5-carboxamide | |
| **30** | 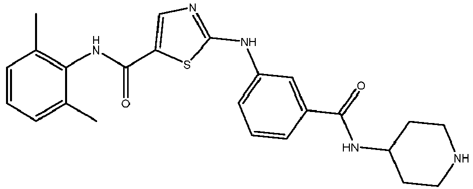 | 1H-NMR(360 MHz, Methanol-d4) δ 8.00 (s, 1H), 7.82 (d, J = 1.8 Hz, 1H), 7.57 (dd, J = 8.2, 2.3, 1.0 Hz, 1H), 7.41 (t, 1H), 7.09 (s, 2H), 7.05 (d, J = 7.6, 1.3 Hz, 2H), 4.59 (d, 1H), 3.83 (d, 1H), 3.19 - 3.06 (m, 2H), 2.87 (s, 1H), 2.23 (s, 6H), 2.08 - 1.78 (m, 2H), 1.45 (s, 2H); m/z [M+H]+ : 450.1 |
| | N-(2,6-dimethylphenyl)-2-((3-(piperidin-4-ylcarbamoyl)phenyl)amino)thiazole-5-carboxamide | |
| **31** | 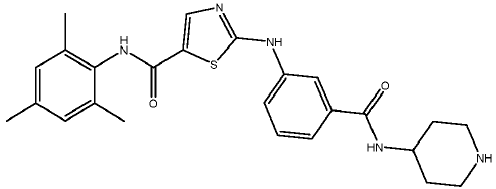 | m/z [M+H]+ : 464.1 |
| | N-mesityl-2-((3-(piperidin-4-ylcarbamoyl)phenyl)amino)thiazole-5-carboxamide | |
| **32** | 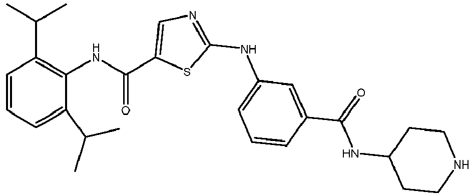 | 1H-NMR(360 MHz, Methanol-d4) δ 8.00 (s, 1H), 7.83 (t, J = 1.9 Hz, 1H), 7.57 (ddd, J = 8.2, 2.3, 1.0 Hz, 1H), 7.41 (s, 1H), 7.31 - 7.24 (m, 1H), 7.18 (d, J = 7.2 Hz, 2H), 7.06 (s, 1H), 4.58 (s, 1H), 3.82 (s, 1H), 3.12 (s, 2H), 2.93 (s, 1H), 1.93 (d, J = 44.2 Hz, 2H), 1.46 (s, 2H), 1.25 (s, 1H), 1.18 (d, J = 6.6 Hz, 12H), 1.12 (s, 1H); 13C-NMR(91 MHz, Methanol-d4) δ 172.17, 169.08, 163.26, 148.09, 143.28, 142.13, 137.84, 132.65, 130.64, 129.52, 124.44, 124.28, 121.88, 120.73, 117.41, 49.40, 49.00, 30.00, 24.16, 23.85; m/z [M+H]+ : 505.3 |
| | N-(2,6-diisopropylphenyl)-2-((3-(piperidin-4-ylcarbamoyl)phenyl)amino)thiazole-5-carboxamide | |
| **33** | 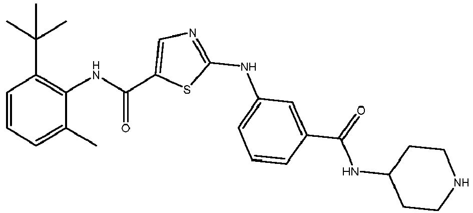 | ¹H NMR (360 MHz, Methanol-*d*₄) δ 8.03 (s, 1H), 7.88 - 7.82 (m, 1H), 7.62 (ddd, *J* = 8.3, 2.3, 1.0 Hz, 1H), 7.44 (t, *J* = 7.9 Hz, 1H), 7.35 (dd, *J* = 6.9, 2.6 Hz, 1H), 7.23 - 7.15 (m, 2H), 7.08 (dt, *J* = 7.6, 1.2 Hz, 1H), 4.58 (d, *J* = 13.3 Hz, 1H), 3.80 (d, *J* = 13.7 Hz, 1H), 3.35 (s, 2H), 3.23 - 3.08 (m, 1H), 2.95 (tt, *J* = 10.8, 4.1 Hz, 2H), 2.24 (s, 3H), 1.96 (s, 1H), 1.83 (d, *J* = 13.2 Hz, 1H), 1.40 (s, 9H); 13C-NMR(91 MHz, Methanol-d4) δ 172.10, 169.12, 163.02, 149.40, 143.35, 142.07, 139.66, 138.05, 135.198, 130.58, 129.65, 128.90, 125.94, 124.65, 121.84, 120.56, 117.43, 49,850, 49.520, 49.00, 36.270, 31.697, 18.746; m/z [M+H]+ : 491.2 |
| | N-(2-(tert-butyl)-6-methylphenyl)-2-((3-(piperidin-4-ylcarbamoyl)phenyl)amino)thiazole-5-carboxamide | |
| **34** | 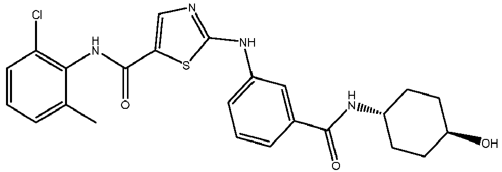 | ¹H NMR (400 MHz, DMSO-d6) δ 10.76 (s, 1H), 9.95 (s, 1H), 8.23 - 8.12 (m, 2H), 8.05 -8.00 (m, 1H), 7.87 - 7.81 (m, 1H), 7.49 - 7.37 (m, 3H), 7.33 - 7.22 (m, 2H), 4.56 (d, *J* = 4.4 Hz, 1H), 3.79 - 3.65 (m, 1H), 3.44-3.36 (m, 1H), 2.24 (s, 3H), 1.84 (t, *J* = 14.1 Hz, 4H), 1.44 - 1.31 (m, 2H), 1.27 (d, *J* = 10.3 Hz, 2H); ¹³C NMR (126 MHz, DMSO-d6) δ 169.64, 162.41, 143.76, 140.33, 137.26, 134.24, 130.32, 130.15, 129.61, 128.34, 123.73, 122.53, 122.31, 118.46, 70.52, 61.54, 49.93, 47.87, 35.03, 31.45, 20.87, 18.70, 14.47, 9.24; m/z [M+H]+ : 485.2 |
| | N-(2-chloro-6-methylphenyl)-2-((3-(((1r,4r)-4-hydroxycyclohexyl)carbamoyl)phenyl)amino)thiazole-5-carboxamide | |
| **35** | 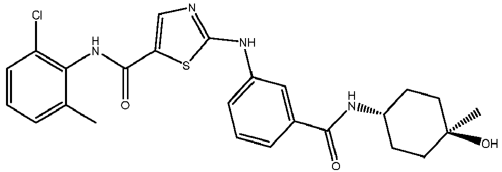 | ¹H NMR (400 MHz, DMSO-d6) δ 10.75 (s, 1H), 9.94 (s, 1H), 8.15 (d, *J* = 6.5 Hz, 2H), 8.01 (t, *J* = 1.8 Hz, 1H), 7.89 - 7.77 (m, 1H), 7.52 - 7.34 (m, 3H), 7.34 - 7.16 (m, 2H), 3.83 - 3.77 (m, 2H), 2.23 (s, 3H), 1.75 - 1.71 (m, 4H), 1.46 (td, *J* = 11.3, 10.2, 3.3 Hz, 4H), 1.15 (s, 3H); ¹³C NMR (126 MHz, DMSO-d6) δ 169.64, 162.41, 143.76, 140.33, 137.26, 134.24, 130.32, 130.15, 129.61, 128.34, 123.73, 122.53, 122.31, 118.46, 70.52, 61.54, 49.93, 47.87, 35.03, 31.45, 20.87, 18.70, 14.47, 9.24; m/z [M+H]+ : 499.2 |
| | N-(2-chloro-6-methylphenyl)-2-((3-(((1r,4r)-4-hydroxy-4-methylcyclohexyl)carbamoyl)phenyl)amino)thiazole-5-carboxamide | |
| **36** | 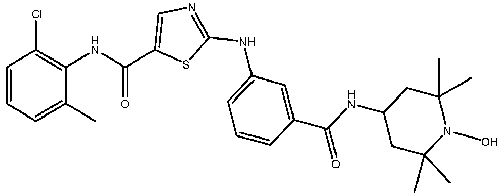 | m/z [M+H]+ : 541.2 |
| | N-(2-chloro-6-methylphenyl)-2-((3-((1-hydroxy-2,2,6,6-tetramethylpiperidin-4-yl)carbamoyl)phenyl)amino)thiazole-5-carboxamide | |
| **37** | 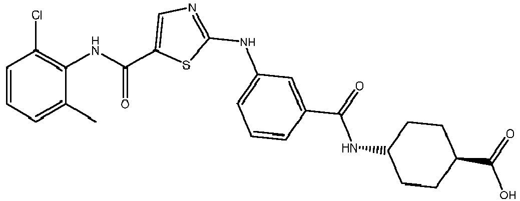 | ¹H NMR (400 MHz, DMSO-d6) δ 10.76 (s, 1H), 9.94 (s, 1H), 8.25 (d, J = 7.9 Hz, 1H), 8.15 (s, 1H), 8.08 - 7.98 (m, 1H), 7.90 - 7.77 (m, 1H), 7.53 - 7.35 (m, 3H), 7.33 - 7.21 (m, 2H), 3.75 - 3.72 (m, 2H), 2.24 (s, 3H), 2.21 - 2.11 (m, 1H), 2.01 - 1.85 (m, 4H), 1.48 - 1.32 (m, 4H); ¹³C NMR (101 MHz, DMSO-d6) δ 166.58, 165.60, 159.15, 156.15, 142.31, 140.32, 138.76, 136.03, 129.02, 128.82, 127.00, 120.65, 119.98, 117.01, 67.97, 64.89, 47.53, 45.85, 37.93, 28.74, 26.38, 25.93, 25.85, 18.27; m/z [M+H]+ : 513.1 |
| | (1r,4r)-4-(3-((5-((2-chloro-6-methylphenyl)carbamoyl)thiazol-2-yl)amino)benzamido)cyclohexane-1-carboxylic acid | |
| **38** | 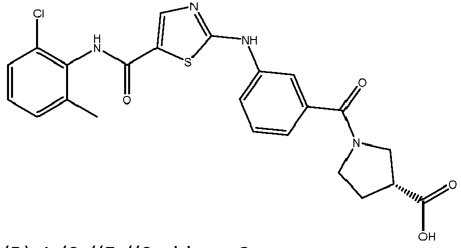 | ¹H NMR (400 MHz, DMSO-d6) δ 10.78 (s, 1H), 9.95 (s, 1H), 8.15 (s, 1H), 7.88 (s, 1H), 7.68 (d, *J* = 8.1 Hz, 1H), 7.42 (td, *J* = 7.9, 7.2, 3.1 Hz, 2H), 7.33 - 7.23 (m, 2H), 7.15 (t, *J* = 6.4 Hz, 1H), 3.80 - 3.62 (m, 1H), 3.59 (d, J = 7.0 Hz, 1H), 3.50 (s, 3H), 3.12 (dt, *J* = 21.9, 7.1 Hz, 1H), 2.24 (s, 3H), 2.10 (ddq, *J* = 28.0, 21.5, 6.8, 6.3 Hz, 2H); m/z [M+H]+ : 485 |
| | (R)-1-(3-((5-((2-chloro-6-methylphenyl)carbamoyl)thiazol-2-yl)amino)benzoyl)pyrrolidine-3-carboxylic acid | |
| **39** | 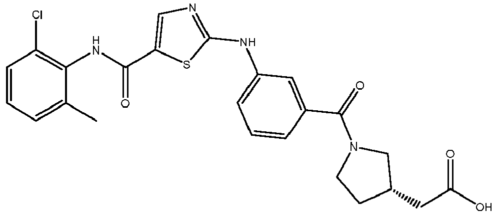 | ¹H NMR (400 MHz, DMSO-d6) δ 12.18 (s, 1H), 10.77 (s, 1H), 9.95 (s, 1H), 8.15 (s, 1H), 7.90 - 7.79 (m, 1H), 7.73 - 7.59 (m, 1H), 7.41 (dtd, *J* = 6.6, 3.9, 1.6 Hz, 2H), 7.32 - 7.24 (m, 2H), 7.15 (dd, *J* = 11.5, 7.6 Hz, 1H), 3.72 (dd, *J* = 12.1, 7.3 Hz, 1H), 3.63 - 3.53 (m, 1H), 3.52 - 3.42 (m, 2H), 3.13 (dd, *J* = 11.3, 8.1 Hz, 1H), 2.42 (t, *J* = 6.8 Hz, 1H), 2.33 (dd, *J* = 7.0, 3.0 Hz, 1H), 2.24 (s, 3H), 2.15 - 1.98 (m, 1H), 1.58 (ddd, *J* = 14.9, 12.1, 8.5 Hz, 1H); m/z [M+H]+ : 499 |
| | (S)-2-(1-(3-((5-((2-chloro-6-methylphenyl)carbamoyl)thiazol-2-yl)amino)benzoyl)pyrrolidin-3-yl)acetic acid | |
| **40** | 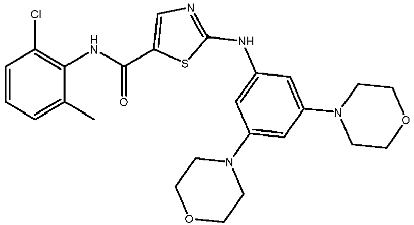 | ¹H NMR (400 MHz, DMSO-d6) δ 9.83 (s, 1H), 8.09 (s, 1H), 7.39 (dd, *J* = 7.5, 2.0 Hz, 1H), 7.32 - 7.19 (m, 2H), 6.65 (s, 2H), 6.21 (s, 1H), 3.84 - 3.62 (m, 8H), 3.15-2.99 (m, 8H), 2.23 (s, 3H); ¹³C NMR (101 MHz, DMSO-d6) δ 167.35, 161.77, 152.56, 141.55, 138.78, 133.47, 129.01, 128.16, 126.99, 122.24, 118.66, 97.79, 97.15, 66.12, 48.71, 18.27; m/z [M+H]+ : 514.2 |
| | N-(2-chloro-6-methylphenyl)-2-((3,5-dimorpholinophenyl)amino)thiazole-5-carboxamide | |
| **41** | 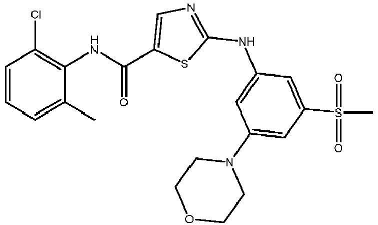 | ¹H NMR (400 MHz, DMSO-d6) δ 9.95 (s, 1H), 8.16 (s, 1H), 7.69 - 7.65 (m, 1H), 7.51 - 7.46 (m, 1H), 7.41 (dd, *J* = 7.5, 2.0 Hz, 1H), 7.33 - 7.23 (m, 2H), 7.09 (t, *J* = 1.8 Hz, 1H), 3.78 (dd, *J* = 6.2, 3.6 Hz, 4H), 3.21 (d, *J* = 3.7 Hz, 7H), 2.24 (s, 3H); ¹³C NMR (101 MHz, DMSO-d6) δ 166.29, 161.60, 159.05, 152.14, 142.41, 142.22, 141.82, 138.77, 133.31, 132.38, 129.04, 128.27, 127.02, 107.36, 106.59, 105.85, 65.88, 47.81, 43.47, 18.25; m/z [M+H]+ : 507.1 |
| | N-(2-chloro-6-methylphenyl)-2-((3-(methylsulfonyl)-5-morpholinophenyl)amino)thiazole-5-carboxamide | |

The compound according to the invention can be used in the treatment of a pathologically increased activity of a protein kinase or the treatment of a pathological binding of a pathogen to a receptor protein kinase of a human being or an animal. The animal may be a mammal. The protein kinase may be a receptor protein kinase or non-receptor protein kinase, such as ABL1 or ABL2. In particular above compound No. 33 has been found to have a high selectivity for inhibition of activity of ABL1 and/or ABL2. The protein kinase may be tyrosine kinase, serine-threonine kinase, or atypical kinase.

In particular the protein kinase may be ephrin receptor (Eph), a tyrosine kinase of the proto-oncogene tyrosine kinase (Src) family, Bruton's tyrosine kinase (BTK), discoidin domain receptor tyrosine kinase (DDR1), discoidin domain-containing receptor 2 (DDR2), a protein kinase of the ErbB family, CSK, RIPK2, SIK2, SIK3, TEC, TNK2, or ADCK3 which is also known as COQ8A. The ephrin receptor may be EphA2. The protein kinase of the ErbB family may be EGF-receptor. The tyrosine kinase of the proto-oncogene tyrosine kinase (Src) family may be SRC, YES1, FYN, FGR, LCK, HCK, BLK, LYN, or FRK. The mentioned kinases are named by the universal protein database UniProt as follows:
SRC : Proto-oncogene tyrosine-protein kinase Src, Proto-oncogene c-Src;
YES1: Tyrosine-protein kinase Yes, Proto-oncogene c-Yes;
FYN: Tyrosine-protein kinase Fyn, Proto-oncogene Syn, Proto-oncogene c-Fyn;
FGR: Tyrosine-protein kinase Fgr, Gardner-Rasheed feline sarcoma viral (v-fgr) oncogene homolog, Proto-oncogene c-Fgr;
LCK: Tyrosine-protein kinase Lck, Leukocyte C-terminal Src kinase, Lymphocyte cell-specific protein-tyrosine kinase;
HCK: Tyrosine-protein kinase HCK, Hematopoietic cell kinase;
BLK: Tyrosine-protein kinase Blk, B lymphocyte kinase;
LYN: Tyrosine-protein kinase Lyn, Lck/Yes-related novel protein tyrosine kinase;
FRK: Tyrosine-protein kinase FRK, FYN-related kinase;
DDR1: Epithelial discoidin domain-containing receptor 1;
DDR2: Discoidin domain-containing receptor 2;
ABL1: Abelson tyrosine-protein kinase 1;
ABL2: Abelson tyrosine-protein kinase 2;
CSK: Tyrosine-protein kinase CSK, C-Src kinase;
RIPK2: Receptor-interacting serine/threonine-protein kinase 2;
SIK2: Serine/threonine-protein kinase SIK2, Salt-inducible kinase 2;
SIK3: Serine/threonine-protein kinase SIK3, Salt-inducible kinase 3;
TEC: Tyrosine-protein kinase Tec;
TNK2: Activated CDC42 kinase 1, Tyrosine kinase non-receptor protein 2;
ADCK3: ADCK3 was renamed to COQ8A (Atypical kinase COQ8A, mitochondrial), aarF domain-containing protein kinase 3, Coenzyme Q protein 8A;

The pathologically increased activity of the protein kinase or the pathological binding of a pathogen to a receptor protein kinase may be accompanied by a cancer, in particular squamous head and neck cancer, lung cancer, in particular non-small cell lung cancer, breast cancer, ovarian cancer, endometrial cancer, pancreatic cancer, glioma, in particular glioblastoma, renal cancer, in particular renal carcinoma, in particular clear cell renal carcinoma, melanoma, bladder cancer, gastric cancer, esophageal cancer, in particular esophageal squamous-cell cancer, colorectal cancer, cervical cancer, primary uveal melanoma, gastric carcinoma, squamous cell carcinoma, in particular laryngeal squamous cell carcinoma or cutaneous squamous cell carcinoma, malignant pleural mesothelioma, multiple myeloma, salivary adenoid cystic carcinoma, leukemia, nasopharnyngeal carcinoma, adenoid cystic carcinoma, or prostate cancer. The pathologically increased activity of the protein tyrosine kinase may also be accompanied by an infection with a virus, bacterium, fungus, parasite, or another pathogen, an autoimmune disease, or an inflammation. The virus may be Kaposi's sarcoma-associated herpesvirus (KSHV), herpesvirus, adenovirus, rhesus monkey rhadinovirus, or Hepatitis C virus. The bacterium may be mycobacterium tuberculosis, enteropathogenic E.coli (EPEC), or chlamydia trachomatis. The parasite may be plasmodium falciparum.

The compound according to the invention may be administered, simultaneously or sequentially, with another treatment of the pathologically increased activity of the protein kinase or the receptor protein kinase and/or a therapy of a pathological condition accompanying the pathologically increased activity of the protein kinase or the receptor protein kinase. The other treatment or the therapy may be a vaccination, an immunotherapy, a therapy with a targeted cytotoxic agent, e. g. Clostridium perfringens enterotoxin (CPE), or an irradiation, e. g. with UV radiation or radioactive radiation.

The compound according to the invention may also be administered, simultaneously or sequentially, with a pharmaceutical compound other than the compound according to the invention. Such a pharmaceutical compound may be an anti-inflammatory agent, an antibiotic, an antifungal, an antiproliferative agent, a chemotherapeutic agent, an immunosuppressant, an anti-cancer agent, a cytotoxic agent or a protein kinase inhibitor, in particular a protein tyrosine kinase inhibitor, other than the compound according to the invention. A simultaneous or sequential treatment can be particularly useful with the protein kinase inhibitor other than the compound according to the invention in case of a resistance vis-à-vis this protein kinase inhibitor in a treatment with this protein kinase inhibitor alone. For example, the simultaneous or sequential treatment can be a treatment together with an EGF-receptor inhibitor, such as gefitinib or erlotinib in case of a resistance vis-à-vis this EGF-receptor inhibitor in a cancer therapy. The inventors found that resistance vis-à-vis gefitinib is dependent on the degree of EphA2 expression and that EphA2 activity is the driving force in the establishment of resistance vis-à-vis gefitinib. Thus, simultaneous or sequential treatment with an EGF-receptor inhibitor and the compound according to the invention can restore sensitivity against this EGF-receptor inhibitor in case of resistance vis-à-vis this EGF-receptor inhibitor, e. g. in the treatment of a cancer, in particular a lung cancer.

The invention also concerns a pharmaceutical composition for use in the treatment of a pathologically increased activity of a protein kinase or a receptor protein kinase or the treatment of a pathological binding of a pathogen to a receptor protein kinase which pharmaceutical composition comprises a pharmaceutically acceptable vehicle or diluent and at least one compound according to the invention. The pharmaceutical composition may also comprise other therapeutic agents or compounds to be administered simultaneously with the compound according to the invention as described above. It may also comprise conventional solid or liquid vehicles or diluents as well as pharmaceutical additives of a type appropriate to the mode of desired administration according to techniques such as those well known in the art of pharmaceutical formulation. The additives may be, e. g. excipients, binders, preservatives, stabilizers, flavors, et cetera.

The compound according to the invention may be administered by any suitable means, for example, orally, such as in the form of tablets, capsules, granules or powders; sublingually; buccally; parenterally, such as by subcutaneous, intravenous, intramuscular, or intrasternal injection or infusion techniques (e.g., as sterile injectable aqueous or non-aqueous solutions or suspensions); nasally such as by inhalation spray; topically, such as in the form of a cream or ointment; rectally such as in the form of suppositories; or in dosage unit formulations containing non-toxic, pharmaceutically acceptable vehicles or diluents. The present compound may, for example, be administered in a form suitable for immediate release or extended release. Immediate release or extended release may be achieved by the use of suitable pharmaceutical compositions comprising the present compound, or, particularly in the case of extended release, by the use of a device such as a subcutaneous implant or an osmotic pump. The present compound may also be administered by use of liposomes, nanoparticles, nanocarriers, peptide-carriers, conjugates of an antibody and the compound, hyperbranched polymers, micelles, or nanobubbles, or any other compound known in the art for delivery and in particular targeted delivery of pharmaceutical compounds.

Exemplary compositions for oral administration include suspensions which may contain, for example, microcrystalline cellulose for imparting bulk, alginic acid or sodium alginate as a suspending agent, methylcellulose as a viscosity enhancer, and sweeteners or flavoring agents such as those known in the art; and immediate release tablets which may contain, for example, microcrystalline cellulose, dicalcium phosphate, starch, magnesium stearate and/or lactose and/or other excipients, binders, extenders, disintegrants, diluents and lubricants such as those known in the art. The present compounds may also be delivered through the oral cavity by sublingual and/or buccal administration. Molded tablets, compressed tablets or freeze-dried tablets are exemplary forms which may be used. Exemplary compositions include those formulating the present compound(s) with fast dissolving diluents such as mannitol, lactose, sucrose and/or cyclodextrins. Also included in such formulations may be high molecular weight excipients such as celluloses (avicel) or polyethylene glycols (PEG). Such formulations may also include an excipient to aid mucosal adhesion such as hydroxyl propyl cellulose (HPC), hydroxy propyl methyl cellulose (HPMC), sodium carboxy methyl cellulose (SCMC), maleic anhydride copolymer (e.g., Gantrez), and agents to control release such as polyacrylic copolymer (e.g., Carbopol 934). Lubricants, glidants, flavors, coloring agents and stabilizers may also be added for ease of fabrication and use.

Exemplary compositions for nasal aerosol or inhalation administration include solutions in saline which may contain, for example, benzyl alcohol or other suitable preservatives, absorption promoters to enhance bioavailability, and/or other solubilizing or dispersing agents such as those known in the art.

Exemplary compositions for parenteral administration include injectable solutions or suspensions which may contain, for example, suitable non-toxic, parenterally acceptable diluents or solvents, such as mannitol, 1,3-butanediol, water, Ringer's solution, an isotonic sodium chloride solution, or other suitable dispersing or wetting and suspending agents, including synthetic mono- or diglycerides, and fatty acids, including oleic acid.

Exemplary compositions for rectal administration include suppositories which may contain, for example, a suitable non-irritating excipient, such as cocoa butter, synthetic glyceride esters or polyethylene glycols, which are solid at ordinary temperatures, but liquify and/or dissolve in the rectal cavity to release the drug.

Exemplary compositions for topical administration include a topical carrier such as Plastibase (mineral oil gelled with polyethylene).

The effective amount of a compound of the present invention may be determined by one of ordinary skill in the art, and includes exemplary dosage amounts for an adult human from about 0.1 to about 100 mg/kg of body weight of active compound per day, which may be administered in a single dose or in the form of individual divided doses, such as from 1 to 4 times per day. It will be understood that the specific dose level and frequency of dosage for any particular subject may be varied and will depend upon a variety of factors including the activity of the specific compound employed, the metabolic stability and length of action of that compound, the species, age, body weight, general health, sex and diet of the subject, the mode and time of administration, rate of excretion, drug combination, and severity of the particular condition resulting from the pathologically increased activity of the protein kinase or the receptor protein kinase.

The invention also concerns a method for preparing the compound according to the invention, wherein 2-chlorothiazole is reacted with a base and an isocyanatoarene or an isocyanatoheteroarene in a solvent to produce a first intermediate,
wherein the first intermediate is reacted with a 3-substituted aminobenzoic acid ester and a proton donating agent to yield a second intermediate, wherein the second intermediate is reacted with an amine, in particular a primary amine, and a Lewis acid, in particular bis(trimethylaluminum)-1,4-diazabicyclo[2.2.2]octane adduct (DABAL-Me₃), in said or a further solvent,
or
wherein the first intermediate is reacted with a 3-substituted aminobenzoic acid and a proton donating agent to yield a second intermediate, wherein the second intermediate is reacted with an amine, said base or a further base, and a coupling reagent in said or a further solvent. The amine may be a primary or a secondary amine. The amidation reaction of the second intermediate containing a carboxyl group with the amine and the coupling reagent may be performed in two steps as described, e. g. in Montalbetti CAGN, Falque V, Tetrahedron, 2005, 61(46):10827-10852. The first step is an activation of the second intermediate by the coupling reagent. The second step is an aminolysis of the activated second intermediate by the amine
or
wherein the first intermediate is reacted with a 3,5-substituted aniline and a proton donating agent.

The base may be a strong base, i. e., a base that is completely dissociated during the reaction of the 2-chlorothiazole with the base and the derivative of isocyanatobenzene in the polar aprotic solvent.

If there is a functional group which requires protection during the preparation of the compound, deprotection may be required for producing the final compound. A functional group requiring protection may be an amine group which could be protected by a tert-butyloxycarbonyl moiety. It may be deprotected by acid deprotection for releasing the amine..

The 3-substituted aminobenzoic acid may be prepared by a coupling reaction from a halogen substituted or a pseudohalogen substituted 3-aminobenzoic acid by utilizing a catalyst, in particular Pd(PPh₃)₄ or Pd(PPh₃)₄ with a ligand and/or a co-catalyst, in said, the further, or another solvent.

The base may be n-butyllithium. Said, the further, and/or the other solvent may be each independently a polar aprotic solvent, in particular tetrahydrofuran (THF) or dimethylformamide (DMF).

The aminobenzoic acid ester derivative may be ethyl-3-aminobenzoate or 3-amino-N-methylbenzamide. Said 3-aminobenzoic acid derivative may be 3-amino-5-(trifluoromethyl)benzoic acid or 2-aminobenzeneboronic acid. Said proton donating agent may be (+)-camphor-10-sulfonic acid or hydrogen chloride.

The coupling reagent is a usual coupling reagent known in the art. It may be bromotripyrrolidinophosphonium hexafluorophosphate (PyBroP) or O-(7-azabenzotriazol-1-yl)-N,N,N',N'-tetramethyluronium hexafluorophosphate (HATU).

In the following the invention is illustrated by means of examples.
- Fig. 1: shows a schematic diagram of a possible synthesis of a compound according to the invention.
- Fig. 2: shows a schematic diagram of an alternative possible synthesis of a compound according to the invention.
- Fig. 3: shows a schematic diagram of a further alternative possible synthesis of a compound according to the invention.
- Fig. 4: shows graphically the selectivity of each of several inhibitors for EphA2 (CATDS (EphA2) value) in relation to the affinity of this inhibitor to EphA2.
- Fig. 5: shows graphically for each of several inhibitors the CATDS (EphA2) value in relation to the number of kinases that are bound by each of the several inhibitors.
- Fig. 6: shows relative expression and apparent K_{d}-values (K_{d}^{app}) of several cellular proteins of cells of glioblastoma cell line SF-268.
- Fig. 7: shows the effect of siRNA knockdown of EphA2 expression on viability of SF-268 cells.
- Fig. 8: shows the effect of dasatinib and several compounds according to the invention on viability of SF-268 cells.

### 1. General synthesis of a compound according to the invention

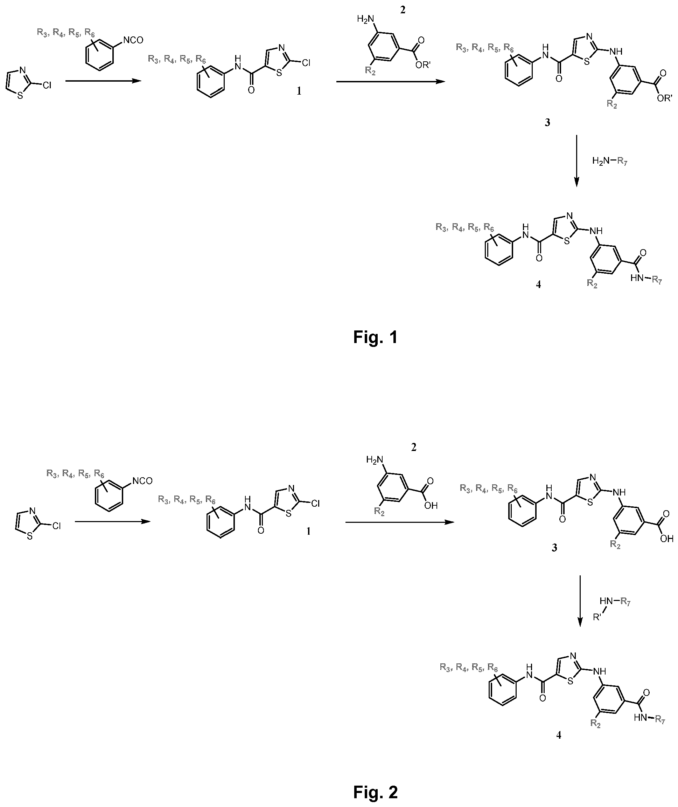

A compound of the invention can be prepared as outlined in Fig. 1 by reacting 2-chlorothiazole with a base, such as n-butyllithium, and an isocyanatoarene or isocyanatoheteroarene in a solvent, such as THF, to produce compound (1). Compound (1) can further be reacted with a 3-substituted aminobenzoic acid ester (2), such as ethyl-3-aminobenzoate or 3-amino-N-methylbenzamide, and a proton donating agent, such as (+)-camphor-10-sulfonic acid, to yield compound (3). Compound (3) can be reacted with a primary amine and a Lewis acid such as DABAL-Me₃ in a solvent, such as THF, to produce compound (4).

A compound of the invention can also be prepared as outlined in Fig. 2 by reacting 2-chlorothiazole with a base, such as n-butyllithium, and an isocyanatoarene or isocyanatoheteroarene in a solvent, such as THF, to produce compound (1). Compound (1) can further be reacted with a 3-substituted aminobenzoic acid (2), such as 3-amino-5-(trifluoromethyl)benzoic acid or 2-aminobenzeneboronic acid, and a proton donating agent, such as (+)-camphor-10-sulfonic acid, to compound (3). Reacting compound (3) with a primary or secondary amine under amidation conditions with a usual coupling reagent known in the art, such as PyBroP or HATU, in a solvent, such as DMF, produces compound (4). If a protecting group, such as tert-butyloxycarbonyl, for protecting a particular reactive functional group, such as an amine, has been introduced, deprotection may be required and performed for producing the final compound (4).

Variations of position R₂ can be achieved in compound (2), (3) or (4) by reacting a halogen or halogen equivalent substituted analogue of this compound under state-of-the-art coupling reaction conditions. For example, compound (2) can be prepared from a halogen or halogen equivalent substituted 3-aminobenzoic acid under a state-of-the-art coupling reaction condition by use of a catalyst, such as Pd(PPh₃)₄, or Bis(diphenylphosphino)ferrocene]dichloropalladium(II) (PdCl₂(dppf)) with or without a metal ligand such as a Buchwald ligand and/or a co-catalyst such as copper(I) iodide for Sonogashira type coupling, in a solvent, such as DMF. The halogen equivalent may be triflate, mesylate or nonaflate. State-of-the-art coupling reaction condition and the catalyst may be anyone known from Heck-, Sonogashira-, Suzuki-, Stille-, Kumada-, Hiyama-, Negishi-, or Buchwald- type cross-coupling reactions. The coupled synthon can be further derivatised. For instance a triazole can be obtained as R₂ if Huisgen-type alkyne-azide cycloaddition is used after introducing an ethynyl moiety as R₂ by an appropriate coupling reaction such as Sonogashira coupling with for instance trimethylsilylacetylene. R₂ can also be a substituted alkene if, for instance, alkene cross-metathesis reaction is used after introduction of a vinyl by an appropriate coupling reaction such as Stille-type reaction with for instance tributyl(vinyl)tin. Alternatively the halogen or halogen equivalent can be exchanged by a metal in a catalyzed or non-catalyzed process for further derivatization. For instance, lithium-halogen exchange, Knochel-type chemistry, Miyaura-type pallladium catalysed borylation can be performed.

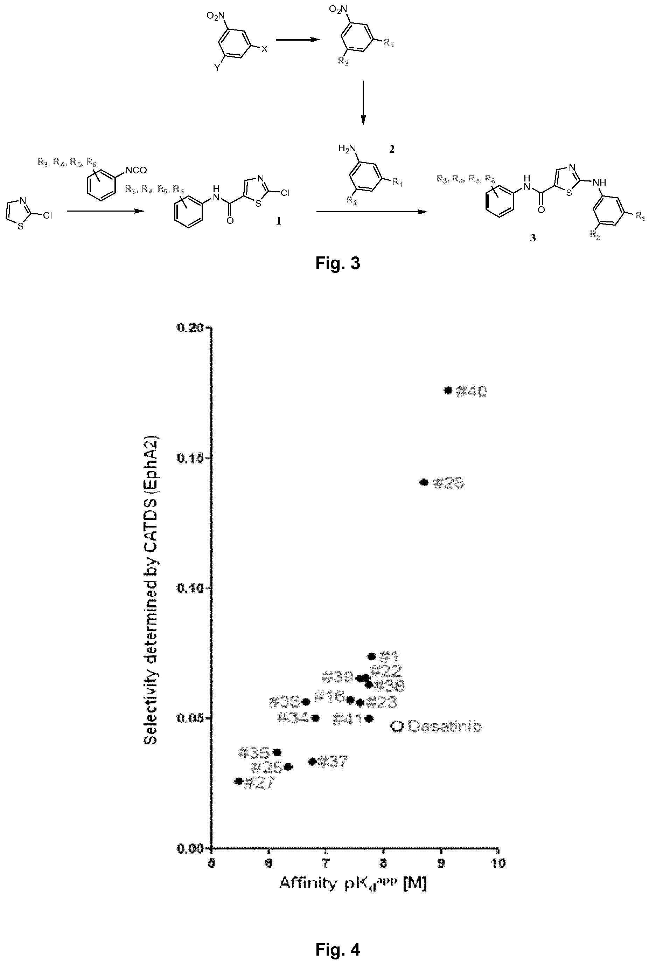

A compound of the invention can further be prepared as outlined in Fig. 3 by reacting 2-chlorothiazole with a base, such as n-butyllithium, and a isocyanatoarene or isocyanatoheteroarene in a solvent, such as tetrahydrofuran, to produce compound (1) as first intermediate. Compound (1) can further be reacted with a 3,5-substituted aniline (2), such as 3,5-dimorpholinoaniline or 3-(methylsulfonyl)-5-morpholinoaniline, and a proton donating agent, such as HCl, in a solvent, such as isopropanol, to compound (3).

Compound (2) may be prepared by substitution of a halogenated nitrobenzene in a solvent (such as dimethylformamide or DMSO) and subsequent reduction to the 3,5-substituted aniline using hydrogen and a catalyst, such as palladium on activated charcoal (Pd-C), in a solvent, such as ethanol.

If there is a functional group, which requires protection, deprotection may be required and performed for producing the final compound.

### 2. Synthesis of specific compounds according to the invention

### a) Synthesis of compound No. 1 according to the above table

A solution of 2-chlorothiazole (717 mg, 6.0 mmol) in THF (10 ml), under argon, was cooled to -78°C. A 2.5 M solution of n-butyllithium in hexanes (2.64 ml, 6.6 mmol) was added dropwise. The reaction mixture was stirred at -78°C for 15 min.

A solution of 2-chloro-6-methylphenyl isocyanate (899 µl, 6.6 mmol) in THF (5 ml) was then added. The reaction mixture was kept at -78°C for 2 h and subsequently quenched with a solution of saturated aqueous NH₄Cl (10 ml). The reaction mixture was extracted with ethyl acetate (30 ml). The resulting organic layer was washed with saturated aqueous NaCl (25 ml), dried over MgSO₄ and concentrated in vacuo. The obtained crude was purified by crystallization: petrol ether was added to a solution of the crude in ethyl acetate to yield 2-chloro-N-(2-chloro-6-methylphenyl)thiazole-5-carboxamide (1330 mg, 77%) as a pale yellow solid.

2-Chloro-N-(2-chloro-6-methylphenyl)thiazole-5-carboxamide (230 mg, 0.8 mmol), ethyl 3-aminobenzoate (143 µl, 0.96 mmol) and (+)-camphor-10-sulfonic acid (186 mg, 0.8 mmol) were dissolved in isopropanol (1.5 ml) and irradiated with a microwave synthesis reactor at 120°C for 3 hours under argon and constant stirring. The reaction mixture was diluted with ethyl acetate (25 ml), washed with saturated aqueous NaHCO₃ (25 ml) and saturated aqueous NaCl (25 ml). The combined aqueous layers were further extracted with ethyl acetate (2 * 25 ml). The combined organic fractions were dried over MgSO₄ and concentrated in vacuo. Dichloromethane (DCM) (2 ml) was added to the crude material and the obtained solid was collected by filtration and washed with DCM (10 ml) to yield ethyl 3-((5-((2-chloro-6-methylphenyl)carbamoyl)thiazol-2-yl)amino)benzoate (220 mg, 66%) as a bright white solid.

DABAL-Me₃ (123 mg, 0.48 mmol) was suspended in THF (0.5 ml) and 4-aminopiperidine (252 µl, 2.4 mmol) was added drop wise at room temperature. The reaction mixture was heated to 40°C and stirred for 30 min under argon. Ethyl 3-((5-((2-chloro-6-methylphenyl)carbamoyl)thiazol-2-yl)amino)benzoate (100 mg, 0.24 mmol) was dissolved in THF (1 ml) and added to the reaction tube. The mixture was further irradiated with a microwave synthesis reactor at 120°C for 2 hours, then quenched with saturated aqueous NaHCO₃ (25 ml) and extracted with a solution of 10% (2 N NH₃ in MeOH) in DCM (2 * 25 ml). The combined organic phase was dried over MgSO₄ and concentrated in vacuo. DCM (1 ml) was added to the crude material and the obtained solid was collected by filtration and washed with DCM (10 ml) to yield N-(2-chloro-6-methylphenyl)-2-((3-(piperidin-4-ylcarbamoyl)phenyl)amino)thiazole-5-carboxamide (50 mg, 44%) as a yellow solid.

Compounds No. 1 and 3 to 18 according to the above table were prepared following the preparation scheme according to Fig. 1 by performing the exemplified reaction with 3-substituted aminobenzoic acid ester derivatives and amines. Compound No. 2 is an ester corresponding to compound (3) in Fig. 1. Therefore, no reaction with an amine is performed at the end of synthesis of compound No. 2.

### b) Synthesis of compound No. 19 according to the above table

2-Chloro-N-(2-chloro-6-methylphenyl)thiazole-5-carboxamide was synthesized as described above for synthesis of compound No. 1. 2-Chloro-N-(2-chloro-6-methylphenyl)thiazole-5-carboxamide (1.33 g, 4.63 mmol) and (+)-camphor-10-sulfonic acid (CSA) (1.08 g, 4.63 mmol, dried with toluene) were dissolved in t-butanol (10 ml) and put under argon atmosphere. 3-Amino-5-bromobenzoic acid (1.00 g, 4.63 mmol) was added slowly. The mixture was irradiated with a microwave synthesis reactor at 120 °C for 3.5 h under constant stirring. It was diluted with ethyl acetate (EA) (10 ml), washed with saturated aqueous NH₄Cl (20 ml), washed with saturated aqueous NaCl (20 ml) and extracted with EA (3 * 20 ml). The organic layer was dried with MgSO₄ and the solvent was evaporated. For purification, the crude was resolved in EA (5 ml), cooled down and crystallized by adding chilled petrol ether (PE). After filtration, the product 3-bromo-5-((5-((2-chloro-6-methylphenyl)carbamoyl)thiazol-2-yl)amino)benzoic acid (1.98 g, 92 %) was yielded as a brown solid.

3-bromo-5-((5-((2-chloro-6-methylphenyl)carbamoyl)thiazol-2-yl)amino)benzoic acid (200 mg, 0.43 mmol) and 4-aminopiperidine (90.2 µl, 0.86 mmol) were dissolved in DMF (1.5 ml) and cooled to 0 °C. After adding N,N-diisopropylethylamine (DIEA) (149.7 µl, 0.86 mmol) and triethylamine (TEA) (119.8 µl, 0.86 mmol), PyBroP (300.3 mg, 0.64 mmol) was dissolved in DMF (0.5 ml), cooled to 0 °C and also added to the reaction mixture. The reaction mixture was quenched after 30 min by adding saturated aqueous NaCl (8 ml). The extraction was done with EA (3 * 10 ml) and 15 % (1.33 M NH₃ in MeOH) in DCM (2 * 10 ml). The organic layer was dried over MgSO₄ and concentrated in vacuo. The crude was purified via flash chromatography, using a gradient over 40 min and UV detection at 254 nm and 320 nm. After evaporating the solvent, 2-((3-bromo-5-(piperidin-4-ylcarbamoyl)phenyl)amino)-N-(2-chloro-6-methylphenyl)thiazole-5-carboxamide (141 mg, 60%) was yielded as a clear yellow solid.

Compounds No. 19 to 39 according to the above table were prepared following the preparation scheme according to Fig. 2 by performing the exemplified reaction with 3-substituted aminobenzoic acid derivatives and amines. In case of compound No. 20 HATU was used as coupling reagent and in case of compounds No. 19 and 21 to 39 PyBroP was used as coupling reagent.

### c) Synthesis of compound (3) according to Fig. 2 and below formula (II) for synthesis of compound No. 21 according to the above table

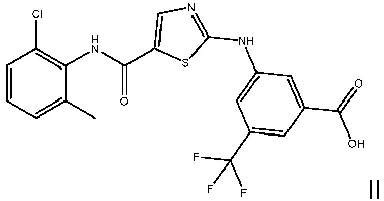

A solution of 2-chlorothiazole (717 mg, 6.0 mmol) in THF (10 ml), under argon, was cooled to -78°C. A 2.5 M solution of n-butyllithium in hexanes (2.64 ml, 6.6 mmol) was added dropwise. The reaction mixture was stirred at -78°C for 15 min. A solution of 2-chloro-6-methylphenyl isocyanate (899 µl, 6.6 mmol) in THF (5 ml) was then added. The reaction mixture was kept at -78°C for 2 h and subsequently quenched with a solution of saturated aqueous NH₄Cl (10 ml). The reaction mixture was extracted with ethyl acetate (30 ml). The resulting organic layer was washed with saturated aqueous NaCl (25 ml), dried over MgSO₄ and concentrated in vacuo. The obtained crude was purified by crystallization: petrol ether was added to a solution of the crude in ethyl acetate to yield 2-Chloro-N-(2-chloro-6-methylphenyl)thiazole-5-carboxamide (1330 mg, 77%) as a pale yellow solid.

2-Chloro-N-(2-chloro-6-methylphenyl)thiazole-5-carboxamide (100 mg, 0.35 mmol) was dried with toluene and dissolved in t-butanol (2 ml). 3-Amino-5-(trifluoromethyl)benzoic acid (107 mg, 0.52 mmol) and CSA (80.8 mg, 0.35 mmol, dried with toluene) were added before putting the mixture under argon and irradiating it for 3.5 h at 120 °C and constant stirring in a microwave synthesis reactor. The crude was diluted with EA (8 ml), washed with saturated aqueous NH₄Cl (10 ml), washed with saturated aqueous NaCl (10 ml) and extracted with EA (3 * 10 ml). Afterwards the organic layer was dried using MgSO₄ and the solvent was evaporated. For purification, it was resolved in EA (2 ml), cooled down and crystallised by adding chilled PE. Filtration yielded 3-((5-((2-chloro-6-methylphenyl)carbamoyl)thiazol-2-yl)amino)-5-(trifluoromethyl)benzoic acid (45.0 mg, 28%) as a white solid.

### d) Synthesis of compound (3) according to Fig. 2 and below formula (III) for synthesis of compound No. 22 according to the above table

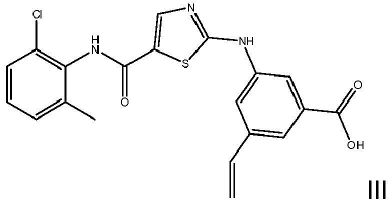

A solution of 2-chlorothiazole (717 mg, 6.0 mmol) in THF (10 ml), under argon, was cooled to -78°C. A 2.5 M solution of n-butyllithium in hexanes (2.64 ml, 6.6 mmol) was added dropwise. The reaction mixture was stirred at -78°C for 15 min. A solution of 2-chloro-6-methylphenyl isocyanate (899 µl, 6.6 mmol) in THF (5 ml) was then added. The reaction mixture was kept at -78°C for 2 h and subsequently quenched with a solution of saturated aqueous NH₄Cl (10 ml). The reaction mixture was extracted with ethyl acetate (30 ml). The resulting organic layer was washed with saturated aqueous NaCl (25 ml), dried over MgSO₄ and concentrated in vacuo. The obtained crude was purified by crystallization: petrol ether was added to a solution of the crude in ethyl acetate to yield 2-Chloro-N-(2-chloro-6-methylphenyl)thiazole-5-carboxamide (1330 mg, 77%) as a pale yellow solid.

2-Chloro-N-(2-chloro-6-methylphenyl)thiazole-5-carboxamide (1.33 g, 4.63 mmol) and (+)-camphor-10-sulfonic acid (CSA) (1.08 g, 4.63 mmol, dried with toluene) were dissolved in t-butanol (10 ml) and put under argon atmosphere. 3-Amino-5-bromobenzoic acid (1.00 g, 4.63 mmol) was added slowly. The mixture was irradiated with a microwave synthesis reactor at 120 °C for 3.5 h under constant stirring. It was diluted with ethyl acetate (EA) (10 ml), washed with saturated aqueous NH₄Cl (20 ml), washed with saturated aqueous NaCl (20 ml) and extracted with EA (3 * 20 ml). The organic layer was dried with MgSO₄ and the solvent was evaporated. For purification, the crude was resolved in EA (5 ml), cooled down and crystallized by adding chilled petrol ether (PE). After filtration, the product 3-bromo-5-((5-((2-chloro-6-methylphenyl)carbamoyl)thiazol-2-yl)amino)benzoic acid (1.98 g, 92 %) was yielded as a brown solid.

3-bromo-5-((5-((2-chloro-6-methylphenyl)carbamoyl)thiazol-2-yl)amino)benzoic acid (100 mg, 0.22 mmol) and tributyl(vinyl)tin (94 µl, 0.32 mmol) were dissolved in dioxane (2.5 ml). Toluene (1.25 ml) was added and the mixture was degassed for 30 min by introducing argon. After adding Pd(PPh₃)₄ (24.8 mg, 0.02 mmol), the reaction was conducted in an oil bath for 4 h at 110 °C under argon atmosphere and constant stirring. Distilled water (5 ml) and saturated aqueous NaCl (5 ml) were used for quenching, EA (3 * 10 ml) for extraction. The organic layer was dried with MgSO₄ before evaporating the solvent. For purification, the crude was resolved in EA (4 ml), cooled down and crystallised by adding chilled PE. Filtration yielded the product 3-((5-((2-chloro-6-methylphenyl)carbamoyl)thiazol-2-yl)amino)-5-vinylbenzoic acid (52.7 mg, 59%) as a pale yellow solid.

### e) Synthesis of compound No. 23 according to the above table

Compound 19 (25 mg, 45.6 µmol), 2-aminobenzeneboronic acid (6.24 mg, 45.6 µmol) and Pd(PPh₃)₄ (5.26 mg, 4.56 µmol) were dissolved in dimethylformamide (0.5 ml) and degassed for 30 min by introducing argon. After adding K₂CO₃ (12.58 mg, 91.2 µmol), the mixture was again put under argon and irradiated in a microwave synthesis reactor for 5 h at 115 °C with constant stirring. Afterwards, it was diluted with methanol (10 ml), filtrated through diatomaceous earth and concentrated in vacuo. Flash silica chromatography purification using a gradient of 0 to 15 % of 1.33 M NH₃ in methanol in dichloromethane and evaporating the solvent yielded compound 23 (8.2 mg, 14.6 µmol, 32.0 %) as a clear yellow solid.

### f) Synthesis of compound No. 40 according to the above table

1,3-difluoro-5-nitrobenzene (5.0 g, 31.43 mmol) and morpholine (10 ml, 115.93 mmol) were brought into solution and sealed into a microwave tube. The reaction mixture was heated to 160 °C for 2 hours in the microwave reactor and cooled to room temperature. The reaction mixture was diluted with ethyl acetate (100 ml), and washed sequentially with saturated aqueous NaCl (3 * 50 ml). The organic layer was dried over Na₂SO₄, filtered and evaporated to afford crude product. The crude product was purified by flash silica chromatography applying a gradient of 0 to 30% ethyl acetate in petroleum ether for elution. Pure fractions were evaporated to dryness to afford 4,4'-(5-nitro-1,3-phenylene)dimorpholine (3.96 g, 43.0 %) as a yellow solid.

Pd-C (0.073 g, 0.68 mmol) and 4,4'-(5-nitro-1,3-phenylene)dimorpholine (2.0 g, 6.82 mmol) in ethanol (50 ml) were stirred under an atmosphere of hydrogen at 20 °C for 12 hours. The reaction mixture was filtered through diatomaceous earth. The solvent was removed under reduced pressure to yield 3,5-dimorpholinoaniline. This product (1.65 g) was used in the next step without further purification.

A solution of 2-chlorothiazole (2.0 g, 16.73 mmol) in tetrahydrofuran (30 ml) was cooled to -78 °C. A 2.5 M solution of n-butyllithium (10 ml, 25.09 mmol) in hexanes was added dropwise to the solution of 2-chlorothiazole. The reaction mixture was stirred at -78 °C for 5 minutes. Afterwards 1-chloro-2-isocyanato-3-methylbenzene (3.36 g, 20.07 mmol) in tetrahydrofuran (10.00 ml) was added slowly. The reaction mixture was stirred at -78 °C for 2 hours. After this time a full conversion was observed. Saturated aqueous NH₄Cl was added and the reaction mixture was extracted with ethyl acetate (2 * 50 ml). The resulting organic layers were combined and dried and concentrated to yield 2-chloro-N-(2-chloro-6-methylphenyl)thiazole-5-carboxamide (3.10 g, 64.5 %) as a white solid.

3,5-dimorpholinoaniline (500 mg, 1.90 mmol) was dissolved in isopropanol (10 ml). 2-chloro-N-(2-chloro-6-methylphenyl)thiazole-5-carboxamide (545 mg, 1.90 mmol) and hydrogen chloride (0.011 ml) were added to the resulting solution. The reaction mixture was stirred overnight at 95° C, diluted with ethyl acetate (50 ml), and washed sequentially with saturated aqueous NaCl (3 * 20 ml). The organic layer was dried over Na₂SO₄, filtered and evaporated to afford crude product. The crude product was purified by C18-flash chromatography using an elution gradient of 0 to 30% acetonitrile in water. Pure fractions were evaporated to dryness to afford N-(2-chloro-6-methylphenyl)-2-((3,5-dimorpholinophenyl)amino)thiazole-5-carboxamide (256 mg, 26.2 %) as a white solid.

### g) Synthesis of compound No. 41 according to the above table

Copper(I) iodide (7.13 g, 37.45 mmol) was added to 1-fluoro-3-iodo-5-nitrobenzene (5.0 g, 18.73 mmol) and methanesulfonic acid sodium salt (3.32 g, 28.09 mmol) in dimethylformamide (50 ml) under nitrogen. The resulting suspension was stirred at 110 °C for 12 hours. The reaction mixture was diluted with ethyl acetate (200 ml), and washed sequentially with saturated aqueous NaCl (3 * 50 ml). The organic layer was dried over Na₂SO₄, filtered and evaporated to afford crude product. The crude product was purified by flash silica chromatography using an elution gradient of 0 to 20% ethyl acetate in petroleum ether. Pure fractions were evaporated to dryness to afford 1-fluoro-3-(methylsulfonyl)-5-nitrobenzene (3.77 g, 92 %) as a yellow solid.

1-fluoro-3-(methylsulfonyl)-5-nitrobenzene (3.67 g, 16.74 mmol) was added to morpholine (30 ml, 347.80 mmol) in dimethylsulfoxide (5 ml) under an atmosphere of nitrogen. The resulting suspension was stirred at 110 °C for 12 hours. The reaction mixture was diluted with ethyl acetate (200 ml) and washed sequentially with saturated aqueous NaCl (3* 50 ml). The organic layer was dried over Na₂SO₄, filtered and evaporated to afford crude product. The crude product was purified by flash silica chromatography using an elution gradient of 0 to 30% ethyl acetate in petroleum ether. Pure fractions were evaporated to dryness to afford 4-(3-(methylsulfonyl)-5-nitrophenyl)morpholine (4.17 g, 87 %) as a yellow solid.

Pd-C (0.074 g, 0.70 mmol) and 4-(3-(methylsulfonyl)-5-nitrophenyl)morpholine (2.0 g, 6.99 mmol) in ethanol (50 ml) were stirred under an atmosphere of hydrogen at 20 °C for 12 hours. The reaction mixture was filtered through diatomaceous earth. The solvent was removed under reduced pressure. The crude product was purified by flash silica chromatography using an elution gradient of 0 to 10% methanol in dichloromethane. Pure fractions were evaporated to dryness to afford 3-(methylsulfonyl)-5-morpholinoaniline (0.860 g, 48.0 %) as a white solid.

A solution of 2-chlorothiazole (2.0 g, 16.73 mmol) in tetrahydrofuran (50 ml) was cooled to -78. A 2.5 M solution of n-butyl lithium (10 ml, 25.09 mmol) in hexanes was added dropwise to the solution of 2-chlorothiazole. The reaction mixture was stirred at -78 °C for 5 minutes. Afterwards 1-chloro-2-isocyanato-3-methylbenzene (3.08 g, 18.40 mmol) in tetrahydrofuran (10 ml) was added slowly. The reaction mixture was stirred at -78 °C for 2 hours. After this time a full conversion was observed. Saturated aqueous NH₄Cl was added and the reaction mixture was extracted with ethyl acetate (2 * 50 ml each). The resulting organic layers were combined and dried and concentrated to yield 2-chloro-N-(2-chloro-6-methylphenyl)thiazole-5-carboxamide (3.60 g, 75.0 %) as a white solid.

Hydrogen chloride (0.011 ml) was added to chloro-N-(2-chloro-6-methylphenyl)thiazole-5-carboxamide (1.680 g, 5.85 mmol) and 3-(methylsulfonyl)-5-morpholinoaniline (1.0g, 3.90 mmol) in isopropanol (30 ml) and warmed to 95 °C. The resulting solution was stirred at 95 °C for 12 hours. The reaction mixture was diluted with ethyl acetate (200 ml) and washed sequentially with saturated aqueous NaCl (3 * 100 ml). The organic layer was dried over Na₂SO₄, filtered and evaporated to afford crude product. The crude product was purified by C18-flash chromatography using an elution gradient of 0 to 30% methanol in water. Pure fractions were evaporated to dryness to afford N-(2-chloro-6-methylphenyl)-2-((3-(methylsulfonyl)-5-morpholinophenyl)amino)thiazole-5-carboxamide (0.675 g, 34.1 %) as a white solid.

### 3. Examination of compounds according to the invention

Several of the compounds according to the invention were further evaluated by a chemical proteomic approach facilitating the identification and quantification of drug-protein interactions. This technology utilizes an affinity matrix comprising five immobilized broad spectrum kinase inhibitors for enrichment of ATP-pocket containing proteins (mainly kinases) from complex native cell lysates as described in Bantscheff et al, Nat Biotechnol., 2007, 25(9):1035-44 and Médard et al, L Proteome Res., 2015, 14(3):1574-86. Coupled to quantitative mass spectrometry readout, this chemical proteomic approach allows for the simultaneous identification and quantification of about 300 native human protein kinases. This affinity matrix was used in competition experiments to determine binding affinities and selectivity profiles of the ATP-competitive small molecule inhibitors according to the invention. For this purpose, the cell lysate was pre-treated with increasing concentrations of one of the compounds prior to affinity based enrichment of protein kinases.

The compounds according to the invention were tested in nine different concentrations (including a DMSO control) ranging from 3 nM to 30 µM. Compounds according to the invention showed binding to target proteins within the Eph receptor family, Src family, Abl family and other protein kinases. Compound selectivity was determined by calculation of a selectivity score named CATDS (concentration and target dependent selectivity). This score measures the binding of a specific protein target at a particular compound concentration relative to all target protein bindings of that compound at the same concentration. It is calculated by dividing the target binding of the target protein (in this case EphA2) by the sum of all target bindings (including the target protein of interest, in this case EphA2) at a particular concentration (in this case the concentration corresponding to the K_{d}^{app} value of a compound for EphA2). Information about the target binding at any concentration were derived from dose response curve fits. As shown in Fig. 4, most of the compounds according to the invention showed nanomolar binding affinities for the protein tyrosine kinase EphA2. Additionally, compound 40 and 28 were found to have an improved selectivity for EphA2 binding in comparison to dasatinib.

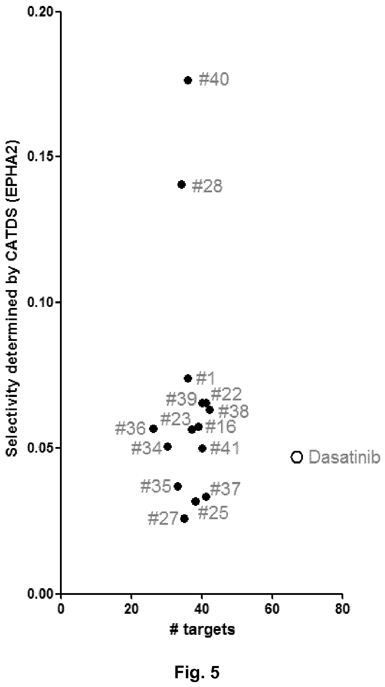

The number of total target proteins targeted by the compounds according to the invention ranged from 26 to 42. This means that the number of total target proteins was reduced for all of the compounds according to the invention compared to dasatinib which targets 67 target proteins. The number of target proteins targeted by indicated compounds according to the invention and dasatinib are shown in Fig. 5.

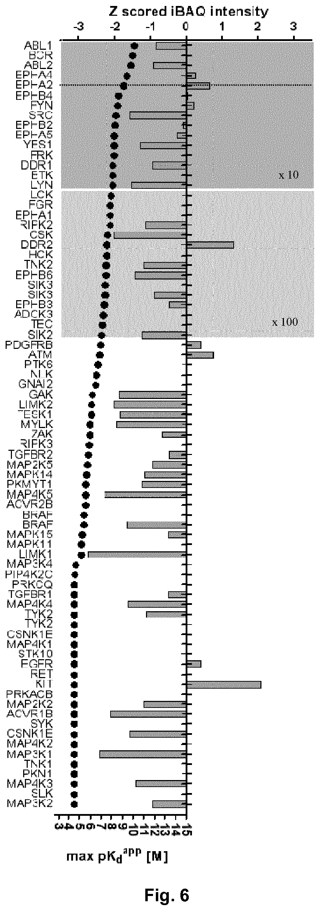

In order to evaluate the cellular effect of the compounds according to the invention, a suitable cell line was selected from the NCI60 panel of cell lines. Above mentioned affinity matrix was used to obtain the differential kinase expression profiles of the NCI60 panel which is a panel of 60 cell lines widely used for cancer research. The relative intensity of each protein was determined for each cell line in comparison to the mean expression value found over all cell lines (z-transformation). Particularly those proteins that are targeted by the compounds according to the invention were taken into account, ranked according to their maximum apparent binding affinity (max pK_{d}^{app}) as determined by selectivity profiling, and theoretical therapeutic windows were set based on the maximum binding affinity for the kinase EphA2 (therapeutic window of 10 or 100). Fig. 6 shows the result for cells of cell line SF-268. SF-268 cells are characterized by high EphA2 expression and comparably low expression of other high affinity off-target proteins targeted by the compounds according to the invention.

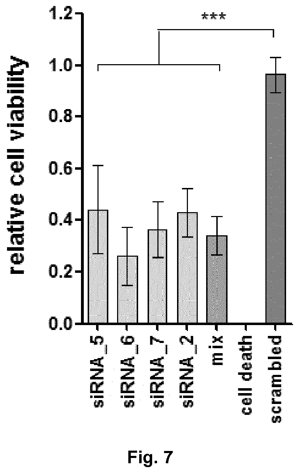

The dependency of viability of SF-268 cells on EphA2 expression was validated by siRNA knockdown experiments. SF-268 cells were treated with four siRNAs targeting EphA2 expression independently and a mixture of the four siRNAs. Experiments were performed in three biological and three technical replicates. Cell death siRNA and scrambled siRNA were used as positive and negative controls. After six days of treatment, cellular viability was determined by a resazurin-based assay system measuring the reduction of resazurin as a function of metabolic activity. Results are shown in Fig. 7. Knockdown of EphA2 resulted in a decrease of 60-80% which revealed the dependency of SF-268 cell viability on EphA2 expression.

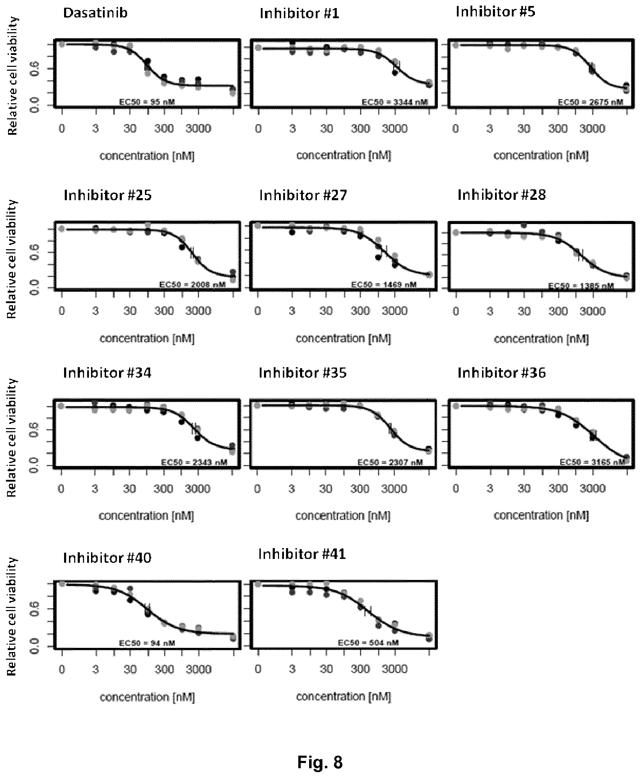

Dasatinib and several of the compounds according to the invention were tested in the EphA2-dependent SF-268 cell line to elucidate the cellular effect of pharmacological inhibition on this cell line. Cellular potency was determined as EC₅₀ value after three days of treatment using the above mentioned resazurin-based assay system in three biological and three technical replicates. Results are shown in Fig. 8. Ten compounds according to the invention resulted in decreased cell viability upon treatment, with potencies ranging from 94 nM to 3.3 µM. Especially, compounds 40 and 41 were found to affect SF-268 cell viability with high cellular potency of 94 nM and 504 nM, respectively.

## Claims

1. Compound, wherein the compound is a substance according to the following formula (I) 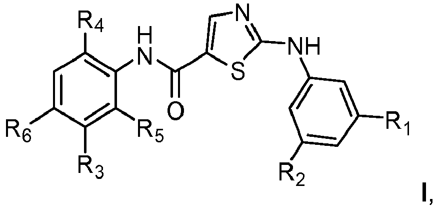
wherein R₁ is 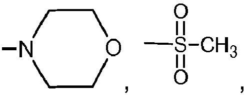 -SO₃H, 1-methyl-4,5-dihydro-1 H-imidazol-2-yl, 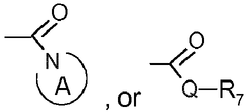 , wherein the ring A is a pyrrolidine, piperidine, morpholine, or imidazole residue or a substituted pyrrolidine, piperidine, morpholine, or imidazole residue, Q is -NH-,-NH-alkyl-, -O-, or -S- and R₇ is -H, a substituted or non-substituted pyrrolidine, piperidine, morpholine, triazine, or imidazole residue, or a substituted or non-substituted alkylpyrrolidine, alkylpiperidine, alkylmorpholine, alkyltriazine, or alkylimidazole residue, or alkyl, alkylester, alkenyl, alkenylester, alkynyl, alkynylester, aminoalkyl, cyanyl, -NO₂, methylsulfonyl residue, or a substituted or non-substituted form of any of the following residues: cycloalkyl, cycloalkylalkyl, cycloalkenyl, cycloalkenylalkyl, bicycloalkyl, bicycloalkylalkyl, bicycloalkenyl, bicycloalkenylalkyl, or another bicyclic residue, aryl, aminoaryl, heteroaryl, heteroaminoaryl, cyclyl, aminocyclyl, heterocyclyl, heteroaminocyclyl, aralkyl, aminoaralkyl, heterocycloalkyl, or heteroaminocycloalkyl,
R₂ is 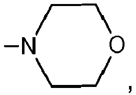 -H, -Cl, -Br, -CF₃, -CH=CH₂ or another alkenyl, -C≡CH or another alkynyl, phenyl or substituted phenyl, triazolyl or another heteroaryl, substituted triazolyl or another substituted heteroaryl, a residue of 9-BBN (9-borabicyclo[3.3.1]nonane), OBBD (9-oxa-10-borabicyclo[3.3.2]decane), trifluoroborate, boronic acid, or an ester of boronic acid and pinacol, methyliminodiacetic acid (MIDA), catechol, pinane diol, ethylene glycol, or propane diol or another boronic acid ester, 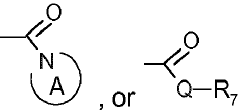 , wherein the ring A is a pyrrolidine, piperidine, morpholine, or imidazole residue or a substituted pyrrolidine, piperidine, morpholine, or imidazole residue, Q is -NH-, -NH-alkyl-, -O-, or -S- and R₇ is -H, a substituted or non-substituted pyrrolidine, piperidine, morpholine, triazine, or imidazole residue, or a substituted or non-substituted alkylpyrrolidine, alkylpiperidine, alkylmorpholine, alkyltriazine, or alkylimidazole residue, alkyl, alkylester, alkenyl, alkenylester, alkynyl, alkynylester, aminoalkyl, cyanyl, -NO₂, methylsulfonyl residue, or a substituted or non-substituted form of any of the following residues: cycloalkyl, cycloalkylalkyl, cycloalkenyl, cycloalkenylalkyl, bicycloalkyl, bicycloalkylalkyl, bicycloalkenyl, bicycloalkenylalkyl, or another bicyclic residue, aryl, aminoaryl, heteroaryl, heteroaminoaryl, cyclyl, aminocyclyl, heterocyclyl, heteroaminocyclyl, aralkyl, aminoaralkyl, heterocycloalkyl, or heteroaminocycloalkyl,
R₃ is -H or -CH₃,
R₄ is -H, -Cl, -Br, -CH₃, -CH(CH₃)₂ or -C(CH₃)₃,
R₅ is -H, -CH₃, -CH(CH₃)₂ or -C(CH₃)₃, and
R₆ is -H or -CH₃,
or a salt of said substance.

2. Compound according to claim 1, wherein R₁ is 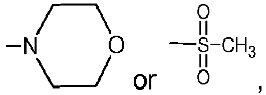 R₂ is 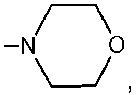 R₃ is -H, R₄ is -Cl and R₅ is -CH₃, -CH(CH₃)₂ or -C(CH₃)₃.

3. Compound according to claim 1, wherein R₁ is 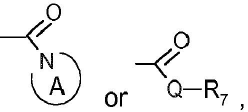 R₂ is -H or -Br, R₃ is -H or -CH₃, R₄ is -H, -Cl, -CH₃, -CH(CH₃)₂ or -C(CH₃)₃ and R₅ is -H, -CH₃, -CH(CH₃)₂ or -C(CH₃)₃ and R₇ is a substituted or non-substituted pyrrolidine, piperidine, triazine, or imidazole residue, or a substituted or non-substituted alkylpyrrolidine, alkylpiperidine, alkyltriazine, or alkylimidazole.

4. Compound according to claim 1, wherein R₁ is 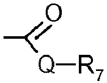 and R₂ is -H, -Br, -CF₃, -CH=CH₂, phenyl or substituted phenyl, wherein R₇ is optionally a substituted or non-substituted pyrrolidine, piperidine, triazine, or imidazole residue, or a substituted or non-substituted alkylpyrrolidine, alkylpiperidine, alkyltriazine, or alkylimidazole.

5. Compound according to claim 1, wherein R₁ is 1-methyl-4,5-dihydro-1H-imidazol-2-yl, R₂ is -H or -Br, R₃ is -H, R₄ is -Cl and R₅ is -CH₃, -CH(CH₃)₂ or -C(CH₃)₃.

6. Compound according to claim 1, wherein said substance is a substance according to any of the following formulae: 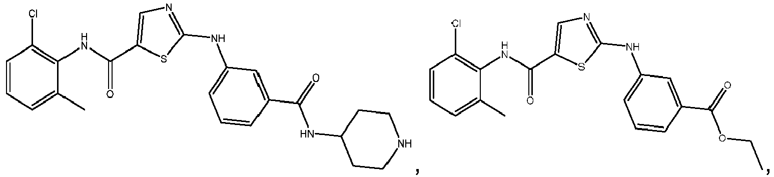 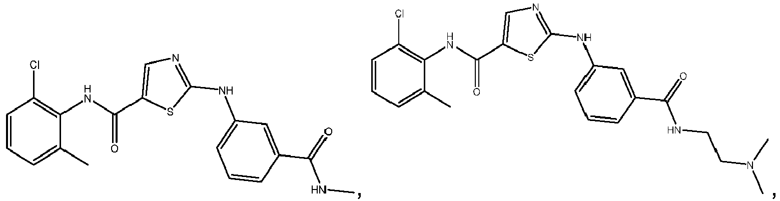 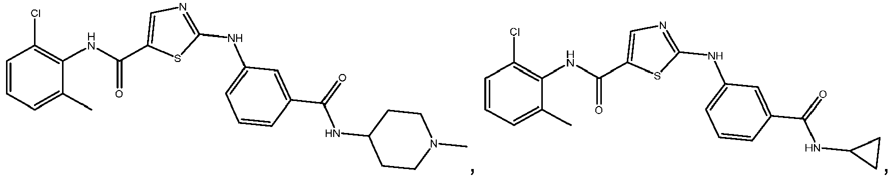 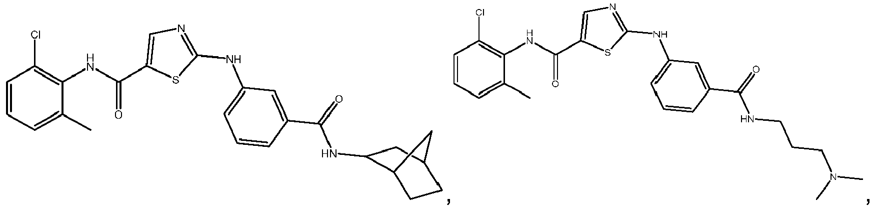 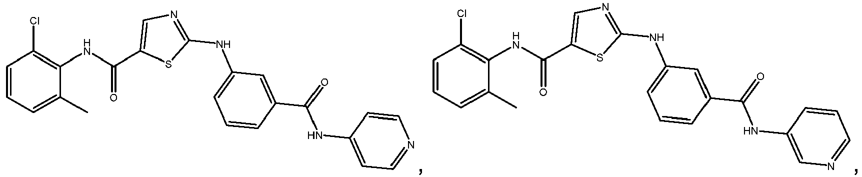 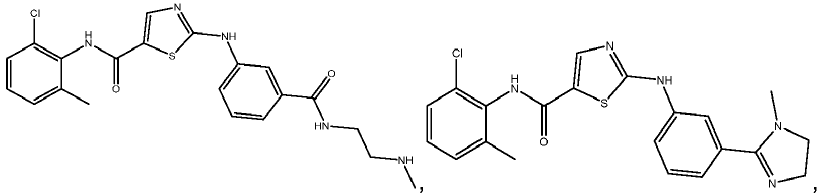 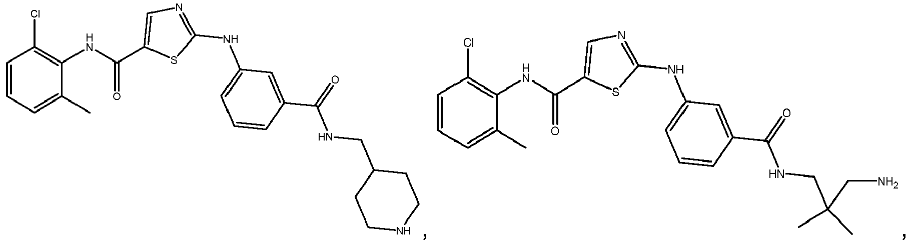 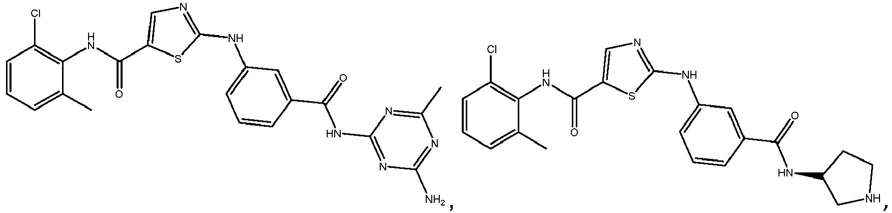 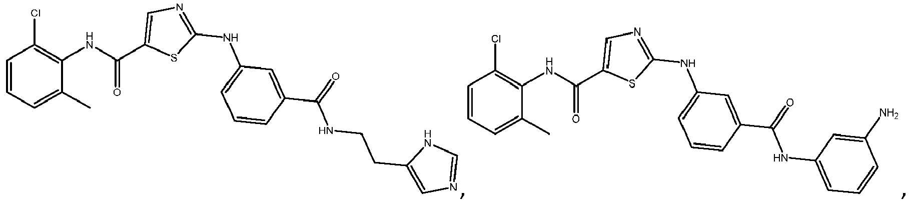 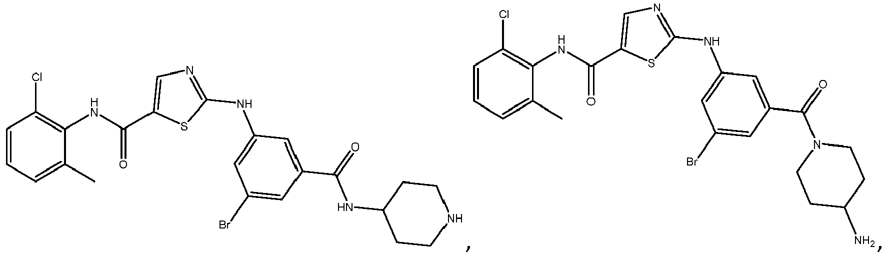 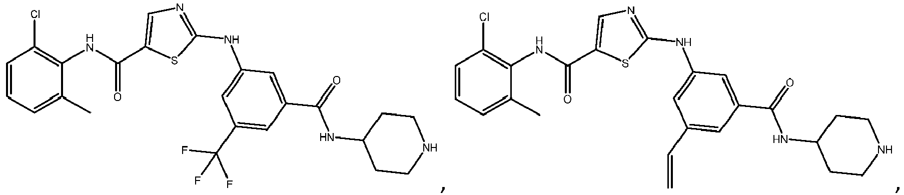 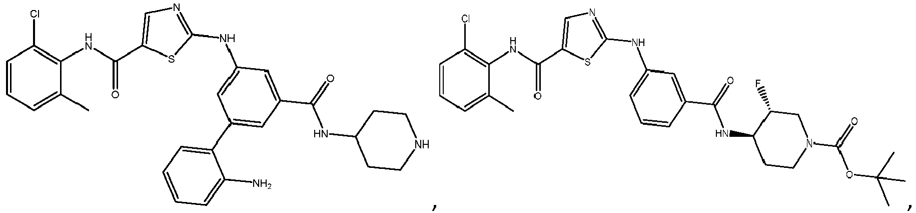 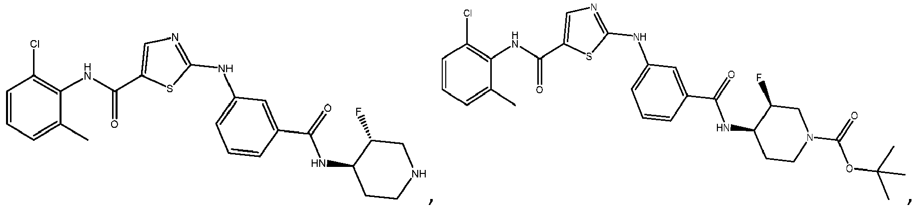 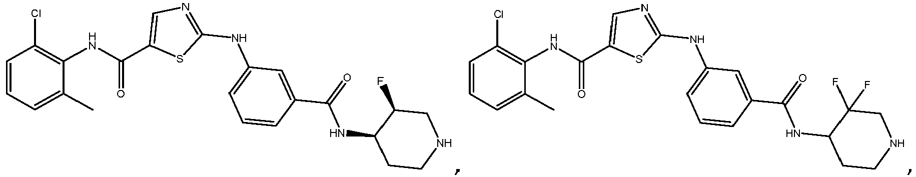 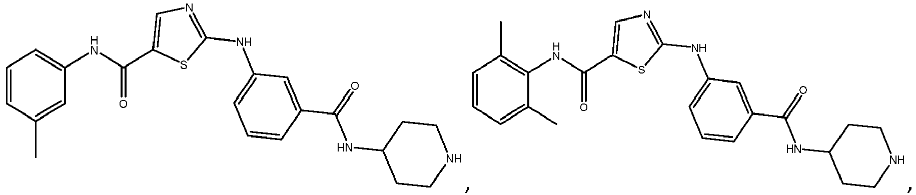 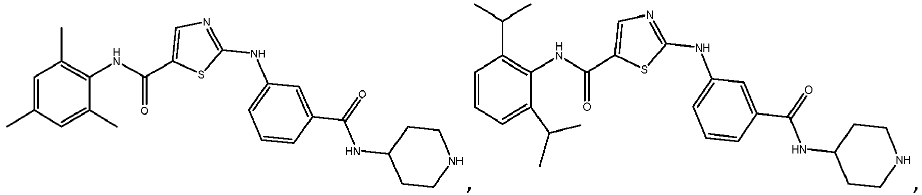 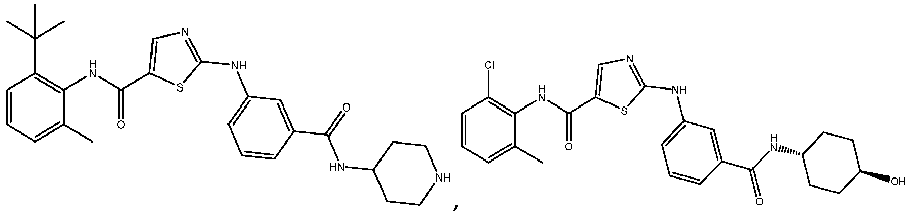 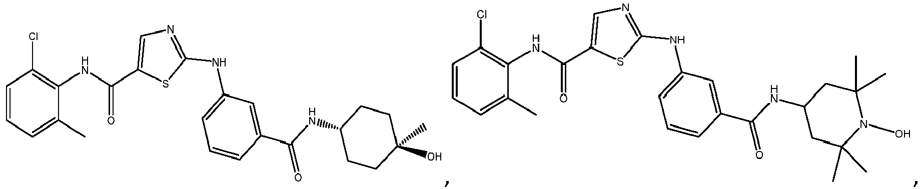 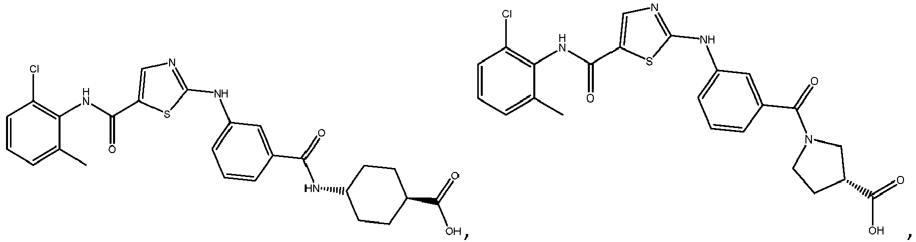 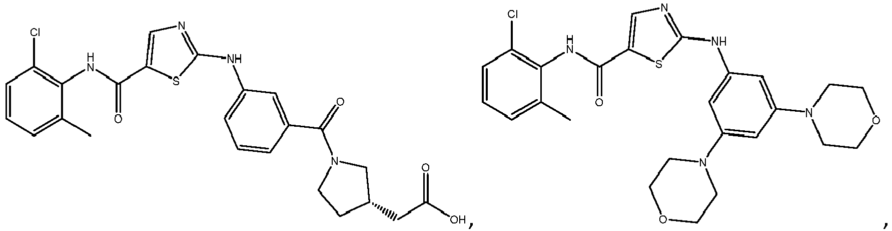 or

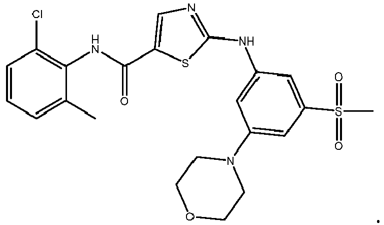

7. Compound according to any of the preceding claims for use in a treatment of a pathologically increased activity of a protein kinase or a receptor protein kinase or a treatment of a pathological binding of a pathogen to a receptor protein kinase of a human being or an animal.

8. Compound according to claim 7 for the use according to claim 7, wherein the protein kinase or receptor protein kinase is tyrosine kinase, serine-threonine kinase, or atypical kinase, in particular ephrin receptor (Eph) or ephrin receptor EphA2, a tyrosine kinase of the proto-oncogene tyrosine kinase (Src) family, Bruton's tyrosine kinase (BTK), discoidin domain receptor tyrosine kinase (DDR1), discoidin domain-containing receptor 2 (DDR2), a protein kinase of the ErbB family, non-receptor tyrosine kinase ABL1 or ABL2, CSK, RIPK2, SIK2, SIK3, TEC, TNK2, or ADCK3, and/or wherein the pathologically increased activity of the protein kinase or the pathological binding of a pathogen to a receptor protein kinase is accompanied by a cancer, an infection with a virus, bacterium, fungus, parasite, or another pathogen, an autoimmune disease, or an inflammation.

9. Pharmaceutical composition for use in a treatment of a pathologically increased activity of a protein kinase or a receptor protein kinase or a treatment of a pathological binding of a pathogen to a receptor protein kinase, which pharmaceutical composition comprises a pharmaceutically acceptable vehicle or diluent and at least one compound of any of claims 1 to 6.

10. Method for preparing the compound of any of claims 1 to 6, wherein 2-chlorothiazole is reacted with a base and an isocyanatoarene or an isocyanatoheteroarene in a solvent to produce a first intermediate,
wherein the first intermediate is reacted with a 3-substituted aminobenzoic acid ester and a proton donating agent to yield a second intermediate, wherein the second intermediate is reacted with an amine and bis(trimethylaluminum)-1,4-diazabicyclo[2.2.2]octane adduct (DABAL-Me3) or another Lewis acid in said or a further solvent,
or
wherein the first intermediate is reacted with a 3-substituted aminobenzoic acid and a proton donating agent to yield a second intermediate, wherein the second intermediate is reacted with an amine, said base or a further base, and a coupling reagent in said or a further solvent
or
wherein the first intermediate is reacted with a 3,5-substituted aniline and a proton donating agent.

11. Method according to claim 10, wherein the 3-substituted aminobenzoic acid is prepared by a coupling reaction from a halogen substituted or a pseudohalogen substituted 3-aminobenzoic acid by utilizing a catalyst, in particular Pd(PPh₃)₄ or Pd(PPh₃)₄ with a ligand and/or a co-catalyst, in said, the further, or another solvent.

12. Method according to claim 10, wherein said base is n-butyllithium.

13. Method according to any of claims 10 to 12, wherein said, the further, and/or the other solvent are each independently a polar aprotic solvent, in particular tetrahydrofuran (THF) or dimethylformamide (DMF).

14. Method according to any of claims 10 to 13, wherein said aminobenzoic acid ester derivative is ethyl-3-aminobenzoate or 3-amino-N-methylbenzamide and said 3-aminobenzoic acid derivative is 3-amino-5-(trifluoromethyl)benzoic acid or 2-aminobenzeneboronic acid and/or wherein said proton donating agent is (+)-camphor-10-sulfonic acid or hydrogen chloride.

15. Method according to any of claims 10 to 14, wherein said coupling reagent is bromotripyrrolidinophosphonium hexafluorophosphate (PyBroP) or O-(7-azabenzotriazol-1-yl)-N,N,N',N'-tetramethyluronium hexafluorophosphate (HATU).
